# EUROPÄISCHE PATENTSCHRIFT

(11) **EP 2 829 244 B1**
(45) Veröffentlichungstag und Bekanntmachung des Hinweises auf die Patenterteilung: **06.12.2017**
(21) Anmeldenummer: 14178310.0
(22) Anmeldetag: 24.07.2014
(51) Int. Cl.: A61B 17/70

(54) **Instrumentensatz für die perkutane Wirbelsäulenstabilisierung mit Hilfe von Pedikelschrauben und Stäben**
Instrument set for percutaneous spinal stabilisation with the aid of pedicle screws and rods
Kit d'instruments pour la stabilisation percutanée de la colonne vertébrale à l'aide de vis pédiculaires et de tiges

(30) Priorität: 26.07.2013 DE 102013108036
(43) Veröffentlichungstag der Anmeldung: 28.01.2015
(73) Patentinhaber: Kraus, Kilian, 97440 Werneck (DE)
(72) Erfinder: Kraus, Kilian, 97440 Werneck (DE)
(74) Vertreter: Schlögl, Markus

(56) Entgegenhaltungen:
- US-A1- 2006 036 255
- US-A1- 2009 228 053
- US-A1- 2012 031 792
- US-A1- 2013 103 039

## Beschreibung

Die Erfindung betrifft einen Instrumentensatz für die perkutane Wirbelsäulenstabilisierung mit Hilfe von Pedikelschrauben und Stäben. Diese Art der Wirbelsäulenstabilisierung wird nicht über einen offenen Schnitt am Rücken vorgenommen, sondern über punktuelle Öffnungen der Haut, wobei für jede zu implantierende Pedikelschraube eine Öffnung erforderlich ist. Zunächst werden mehrere Pedikelschrauben in die rechten und linken Pedikel der Wirbelsäule eingebracht und die Pedikelschrauben der jeweiligen Seite mit einem Stab verbunden. Die Stäbe werden dabei in eine distal, d.h. in einer sich vom Patientenkörper weg weisenden Richtung öffnende Ausnehmung im Kopf der Pedikelschrauben eingesetzt und mit Hilfe von Fixierschrauben fixiert. Wegen der eingeschränkten Zugänglichkeit des Operationsgebietes und der trotz der Anwendung bildgebender Verfahren eingeschränkten visuellen Kontrolle der Operationsdurchführung sind Instrumente für die am offenen Rücken ausgeführte Wirbelsäulenstabilisierung nicht oder allenfalls nur eingeschränkt verwendbar. Ein Instrumentensatz mit den Merkmalen des Oberbegriffs des Anspruchs 1 ist aus der US 2012/031792 A1 bekannt. Die US 2009/228053 A1, die US 2013/103039 A1 und die US 2006/036255 A1 offenbaren weitere Instrumentensätze. Aufgabe der Erfindung ist es, einen Instrumentensatz anzugeben, mit dem eine perkutane Wirbelsäulenstabilisierung sicher und zuverlässig durchführbar ist.
Diese Aufgabe wird durch einen Instrumentensatz nach Anspruch 1 gelöst. Vorteilhafte Weiterbildungen der Erfindung sind Gegenstand der abhängigen Ansprüche. Der Instrumentensatz umfasst einen von einem zentralen Hohlraum axial durchsetzten, eine zylindrische Hülse und einen Schieber aufweisenden Schraubenmanipulator. Die Wand der zylindrischen Hülse ist von zwei diametral gegenüberliegenden, axial verlaufenden und in die proximale Stirnseite der Hülse ausmündenden Fenstern in zwei erste Wandsegmente unterteilt. Die proximalen Enden der ersten Wandsegmente bilden Greifelemente, welche bei einer Schraubenmanipulation zumindest einen Längsabschnitt des Schraubenkopfes zwischen sich aufnehmen. Aufgrund dieser Ausgestaltung lassen sich die Freienden bzw. proximalen Enden der Wandsegmente - auch bei relativ mechanisch fester Auslegung - quasi wie Federzungen zumindest geringfügig radial aufbiegen, so dass der Schraubenmanipulator in Axialrichtung bzw. mit einer in dieser Richtung, also axial verlaufenden Fügebewegung durch eine Haut- bzw. Gewebeöffnung am Rücken des Patienten hindurch und unter Aufweitung der ersten Wandsegmente auf den Kopf aufgesteckt werden kann. Die sich diametral gegenüberliegenden, Greifelemente umgreifen dabei den Kopf, mit anderen Worten ragt der Kopf in einen von den proximalen Enden der ersten Wandsegmente bzw. von den Greifelemente seitlich begrenzten Aufnahmeraum zumindest mit einem Längsabschnitt hinein. Die in Rede stehende radiale Aufweitbarkeit der Greifelemente ist dann besonders vorteilhaft, wenn an den einander zugewandten Innenflächen der Greifelemente ein radial nach innen vorstehendes, insbesondere einstückig mit den Greifelementen ausgebildetes Rast- oder Formschlusselement vorhanden ist, welches in eine vorzugsweise komplementäre Ausnehmung in der Kopfaußenseite eingreift und dabei eine axial und drehfeste Verbindung zwischen Kopf und Schraubenmanipulator gewährleistet.

Ein weiteres Merkmal des Schraubenmanipulators ist der oben genannte Schieber. Er ist in Axialrichtung des Schraubenmanipulators an der Hülse verschiebbar gehalten und so mit den ersten Wandsegmenten verbunden, dass er diese - zumindest in einer proximalen Verschiebe-Endlage gegen ein radiales Auseinanderweichen sichert. Durch diese Ausgestaltung ist gewährleistet, dass ein Schraubenkopf sicher und fest zwischen den Greifelementen eingeklemmt wird. In der proximalen Verschiebe-Endlage des Schiebers kann somit eine Pedikelschraube bzw. deren Kopf mit Hilfe des Schraubenmanipulators mit einer sich quer zur Schraubenlängsachse gerichteten Kraft beaufschlagt und dabei ein Wirbel reponiert, d.h. aus einer dislozierten in eine korrekte Lage gebracht werden.

Weitere vorteilhafte Ausgestaltungen der Erfindung gehen aus der folgenden Beschreibung hervor, die auf die beigefügten Zeichnungen Bezug nimmt. Es zeigen:
Fig. 1A eine Pedikelschraube in Seitenansicht,
Fig. 1B ein Fixierschraube in perspektivischer Darstellung,
Fig. 2 mit Hilfe von Stäben und Pedikelschrauben stabilisierte Wirbelkörper einer Wirbelsäule, wobei die Wirbel in stark schematisierter Form dargestellt sind,
Fig. 3 eine Seitenansicht eines Schraubenmanipulators, der eine Hülse, einen Schieber und eine Verstellmutter umfasst,
Fig. 4 eine Seitenansicht des Schraubenmanipulators, wobei dieser gegenüber Fig. 3 um 90° um seine Mittellängsachse gedreht ist,
Fig. 5 einen Längsschnitt durch den Schraubenmanipulator entsprecht Linie V-V in Fig. 4,
Fig. 6 eine Seitenansicht der Hülse des Schraubenmanipulators,
Fig. 7 eine Seitenansicht der Hülse, gegenüber Fig. 6 um 90° um ihre Mittellängsachse gedreht,
Fig. 8 einen Längsschnitt entsprechend Linie VIII-VIII in Fig. 6,
Fig. 9 eine perspektivische Ansicht des Schiebers des Schraubenmanipulators,
Fig. 10 eine teilweise geschnittene Seitenansicht eines eine Pedikelschraube haltenden Schraubenmanipulators,
Fig. 11 der Schraubenmanipulator von Fig. 10, jedoch um 90° um seine Mittellängsachse gedreht,
Fig. 12 einen Längsschnitt durch den eine Pedikelschraube haltenden Schraubenmanipulator entsprechend Linie XII - XII in Fig. 11,

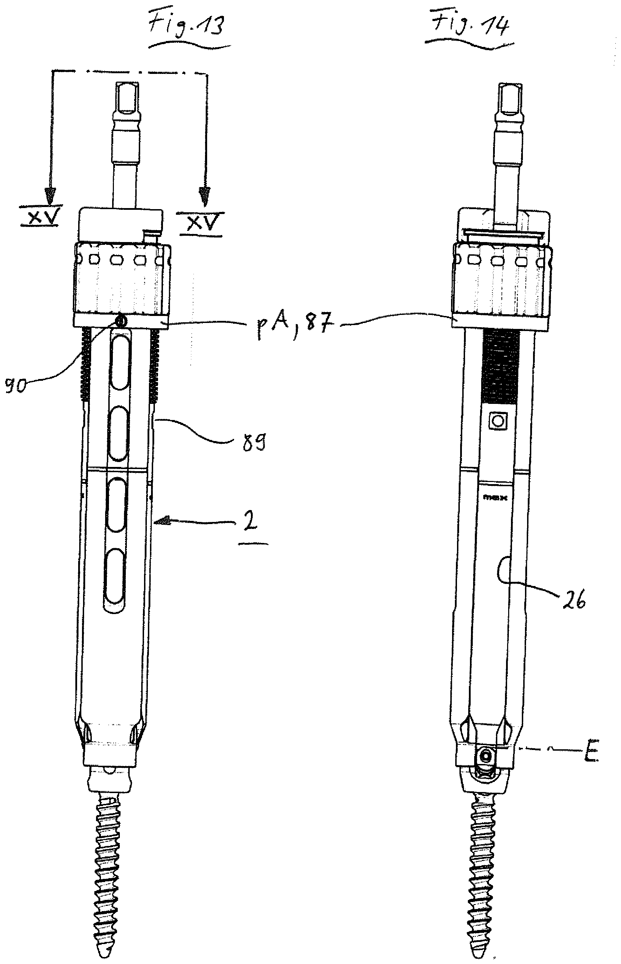

Fig. 13 einen eine Pedikelschraube haltenden Schraubenmanipulator, in den ein Schraubendreher eingesetzt ist,
Fig. 14 den Schraubenmanipulator von Fig. 13, jedoch gedreht um 90° um seine Mittellängsachse,
Fig. 15 einen Längsschnitt entsprechend Linie XV - XV in Fig. 13,
Fig. 16 einen Querschnitt entsprechend Linie XVI - XVI in Fig. 12,
Fig. 17 den Ausschnitt XVII von Fig. 15 in vergrößerter Darstellung,
Fig. 18 die Verstellmutter des Schraubenmanipulators in perspektivischer Darstellung,

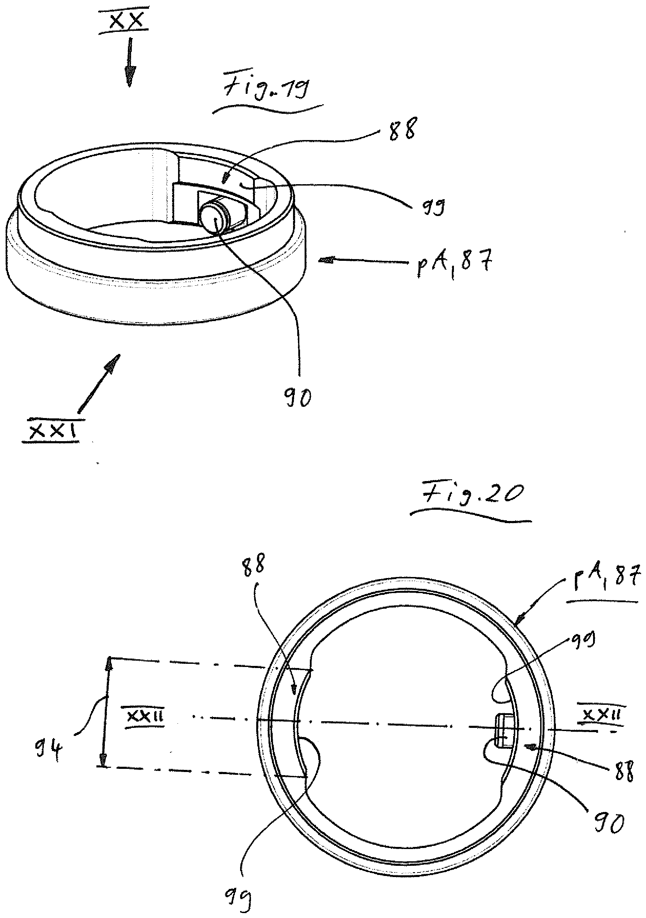

Fig. 19 einen zur axialen Fixierung der Verstellmutter am Schraubenmanipulator dienenden, als Anschlagring ausgebildeten proximalen Anschlag, in perspektivischer Darstellung,
Fig. 20 den Verstellring in einer Draufsicht in Richtung des Pfeils XX in Fig. 19,

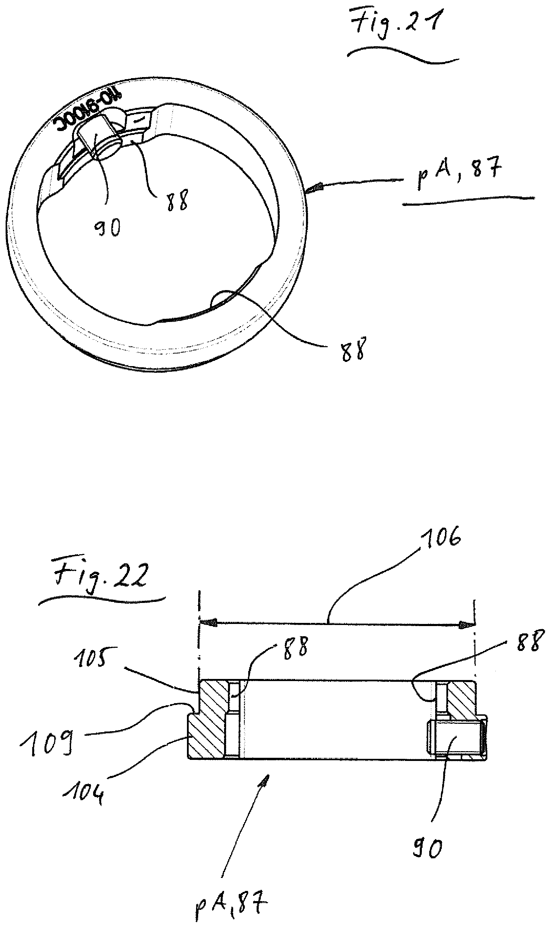

Fig. 21 den Verstellring in Richtung des Pfeils XXI in Fig. 19 gesehen,
Fig. 22 einen Querschnitt entsprechend Linie XXII - XXXII in Fig. 20
Fig. 23 den Schraubendreher in perspektivischer Darstellung,

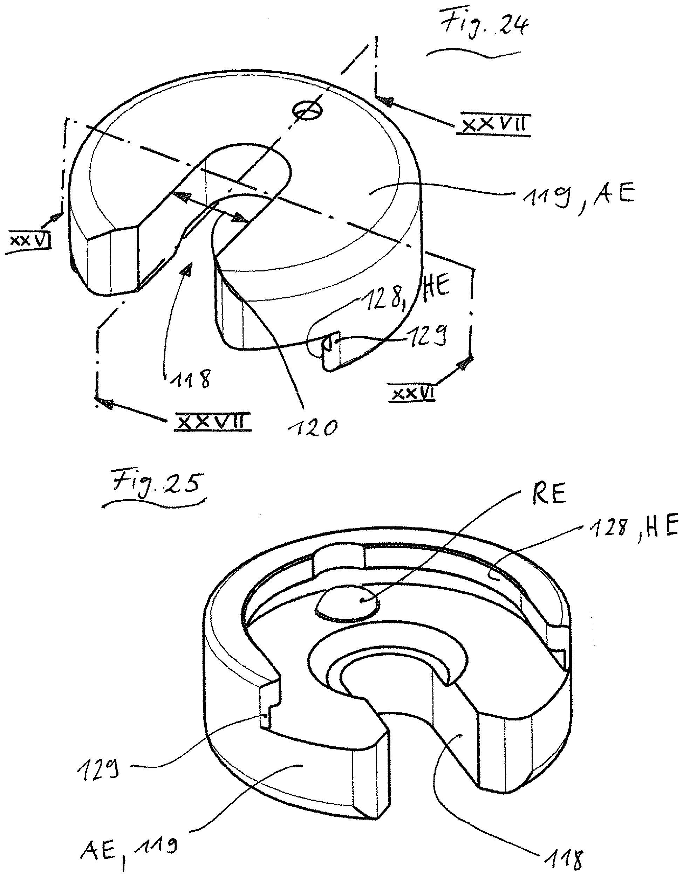

Fig. 24 ein zur axialen Fixierung des Schraubendrehers am Schraubenmanipulator dienendes Verriegelungselement, in perspektivischer Darstellung,
Fig. 25 eine perspektivische Ansicht des Verriegelungselement, jedoch aus einem anderen Blickwinkel gesehen,

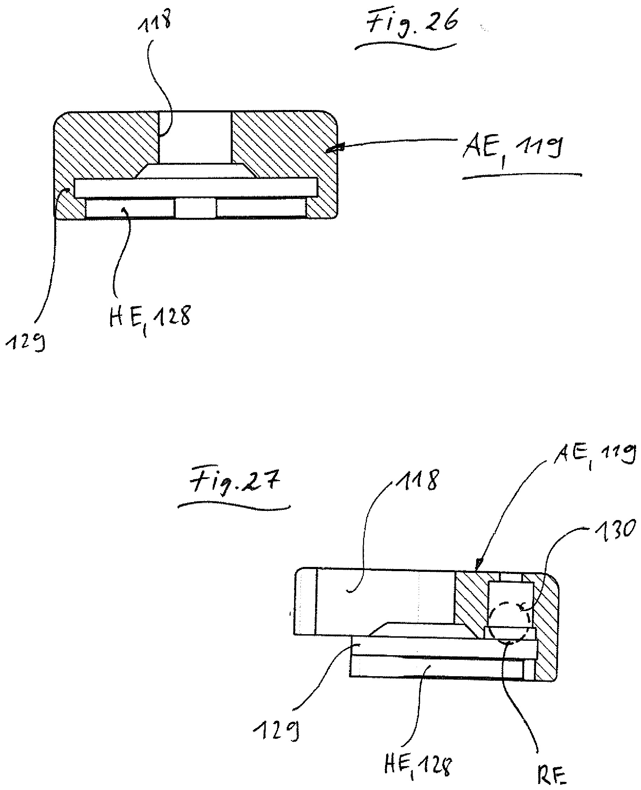

Fig. 26 einen Querschnitt entsprechend Linie XXVI - XXVI in Fig. 24
Fig. 27 einen Querschnitt entsprechend Linie XXVII - XXVII in Fig. 24,
Fig. 28 eine Draufsicht auf die distale Stirnseite der Hülse in Richtung des Pfeils XXVIII in Fig. 6,
Fig. 29 ein zur Handhabung eines Stabs dienendes, ein Antriebs- und ein Abtriebselement umfassendes Greifwerkzeug in Seitenansicht,
Fig. 30 das Greifwerkzeug in Richtung des Pfeils XXX in Fig. 29 gesehen,
Fig. 31 einen Schnitt durch das Greifwerkzeug von Fig. 30, wobei die Schnittebene in der Papierebene verläuft,
Fig. 32 den Ausschnitt XXXIII von Fig. 31 in größerem Maßstab,
Fig. 33 einen Schnitt entsprechend Linie XXXIII - XXXIII in Fig. 31,
Fig. 34 eine Fig. 31 entsprechende Darstellung, wobei sich das Greifwerkzeug jedoch in einer anderen Betriebssituation befindet,
Fig. 35 den Ausschnitt XXXV von Fig. 35 in vergrößertem Maßstab,
Fig. 36 einen Schnitt entsprechend Linie XXXVI - XXXVI in Fig. 34,
Fig. 37 einen ausschnittsweisen Längsschnitt durch das Antriebswerkzeug,
Fig. 38 einen Schnitt entsprechend Linie XXXVIII -XXXVIII in Fig. 30,

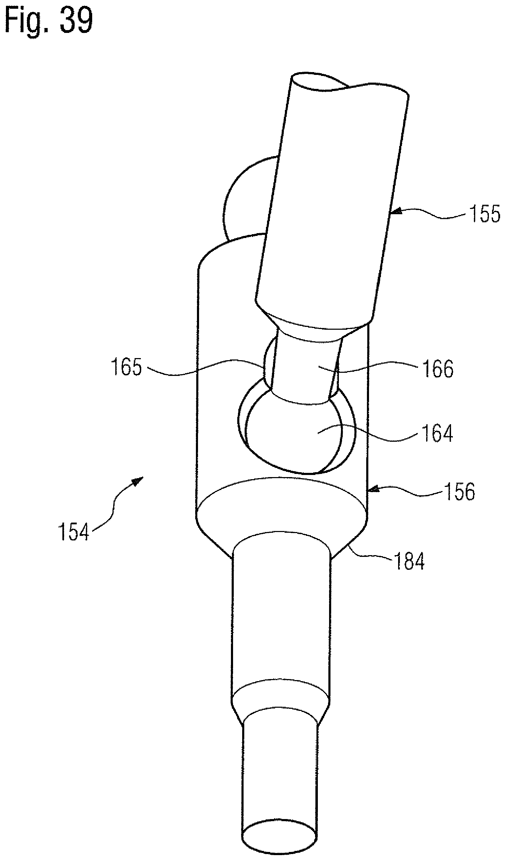

Fig. 39 eine ausschnittsweise perspektivische Darstellung des An- und Abtriebselements,
Fig. 40 den vergrößerten Ausschnitt XL von Fig. 31

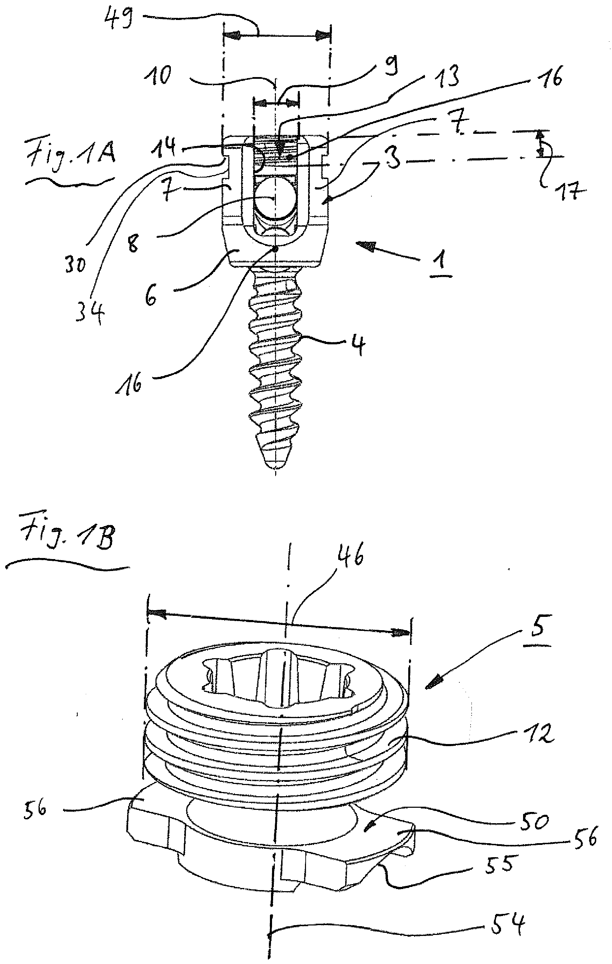

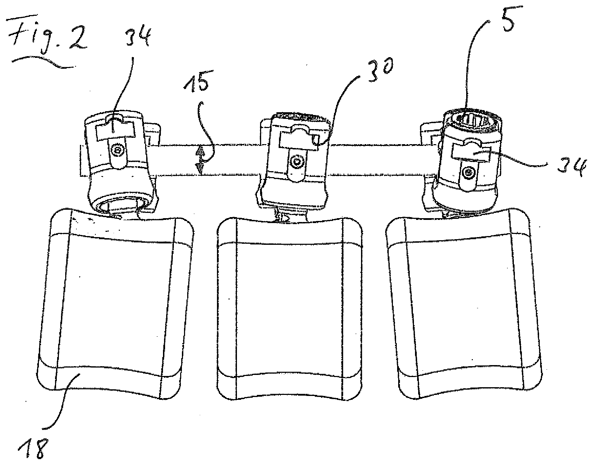

Fig. 1 zeigt ein Beispiel für eine Pedikelschraube 1, die mit Hilfe des erfindungsgemäßen Schraubenmanipulators 2 im Zuge einer perkutanen Wirbelsäulenstabilisierung handhabbar ist. Die Pedikelschraube 1 setzt sich im Wesentlichen aus einem Schraubenkopf 3, einem mit einem Gewinde versehenen Schraubenschaft 4, und einer Fixierschraube 5 zusammen. Der Schraubenkopf 3 weist an seinem dem Schraubenschaft 4 zugewandten unteren Ende eine Kopfbasis 6 auf, an der zwei sich distal erstreckende Wandabschnitte 7 angeformt sind. Wenn hier und weiter unten im Zusammenhang mit Richtungs- und Lageangaben von distal und proximal gesprochen ist, so bezieht sich dies auf die Operationssituation. Die Wandabschnitte 7 liegen sich diametral gegenüber, erstrecken sich jeweils nur über einem Umfangsabschnitt der Kopfbasis 6 und bilden eine seitliche Begrenzung für eine zur Aufnahme eines Stabs 8 dienende, sich in die distale Stirnseite des Schraubenkopfes 3 öffnende Ausnehmung 13. Die Breite 9 der zwischen den sich parallel zur Mittellängsachse 10 des Schraubenkopfes 3 erstreckenden Seitenkanten der Wandabschnitte 7 vorhandenen Zwischenräume 14 entspricht im Wesentlichen dem Durchmesser 15 eines Stabs 8 (Fig. 2) bzw. ist geringfügig größer als dieser.

Der Schraubenschaft 4 ist entweder fest mit dem Schraubenkopf 3 verbunden, um eine Achse 16 schwenkbar am Schraubenkopf 3 gehalten, wie dies bei dem gezeigten Schraubenbeispiel der Fall ist, oder polyaxial am Schraubenkopf 3 gelagert. Die zum Festklemmen des Stabs 8 in der Ausnehmung 13 dienende Fixierschraube 5 ist von der distalen Stirnseite des Schraubenkopfes 3 her in die Ausnehmung 13 eingesetzt. Sie weist etwa die Form einer Madenschraube auf und umfasst ein Außengewinde 12, welches mit einem Innengewinde 16, welches an der Innenseite der Wandabschnitte 7 angeordnet ist, zusammenwirkt. Das Innengewinde 16 erstreckt sich zumindest annähernd vom Freiende der Wandabschnitte 7 in proximaler Richtung weg, wobei seine Gewindelänge 17, also seine axiale Erstreckung, beispielsweise so bemessen ist, dass es mit Axialabstand vor dem Grund der Ausnehmung 13 endet.

Bei einer Wirbelsäulenstabilisierung beispielsweise unter Einbeziehung von drei Wirbelkörpern werden sechs Pedikelschrauben und zwei Stäbe 8 benötigt. Die Pedikelschrauben werden in den rechten und linken Pedikeln der Wirbelkörper 18 mit Hilfe eines in den Schraubenmanipulator axial eingesteckten Schraubendrehers 48, was weiter unten noch näher erläutert wird, eingeschraubt. Die Ausnehmungen 13 der einer Seite zugeordneten Schraubenköpfe 3 sind so ausgerichtet, dass die Zwischenräume 14 alle im Wesentlichen in ein und dieselbe Richtung weisen, was einfacher zu gewährleisten ist, wenn Schraubenkopf 3 und Schraubenschaft 4 gelenkig miteinander verbunden sind. Wenn ein oder mehrere Wirbelkörper 18 des zu stabilisierenden Bereichs der Wirbelsäule disloziert sind, wird mit Hilfe des den Schraubenkopf 3 greifenden Schraubenmanipulators der Wirbelkörper 18 unter entsprechender Schwenkung der Pedikelschrauben 1 reponiert. Auch das Einführen der Stäbe 8 in die Ausnehmungen der Pedikelschrauben 1 wird durch den Schraubenmanipulator erleichtert (siehe weiter unten).

Der Schraubenmanipulator 2 ist von einem zentralen Hohlraum 11 axial durchsetzt und umfasst eine zylindrische Hülse 20 und einen Schieber 24. Der Hohlraum 11 des Schraubenmanipulators ist in Radialrichtung so geformt und bemessen, dass er eine zur Fixierung eines in der distal offenen Ausnehmung 13 einer Pendikelschraube 1 einliegenden Stabs 8 einzuschraubende Fixierschraube 5 aufnehmen kann, d.h. dass diese vom distalen Ende des Schraubenmanipulators 2 her in dessen Hohlraum 11 eingeführt und - etwa mit Hilfe eines Schraubendrehers- zum proximalen Ende des genannten Manipulators transportiert und in die Ausnehmung 13 eingeschraubt werden kann.

Die Wand der Hülse 20 ist von zwei sich diametral gegenüberliegenden, axial verlaufenden und in die proximale Stirnseite der Hülse 20 ausmündenden Fenstern 26 in zwei erste Wandsegmente 27 unterteilt. Die proximalen Enden der ersten Wandsegmente 27 bilden Greifelemente 28, welche bei einer Schraubenmanipulation zumindest einen Längsabschnitt des Schraubenkopfes 3 einer Pedikelschraube 1 zwischen sich aufnehmen und festklemmen. Der Schraubenmanipulator 2 ist in Axialrichtung so bemessen, dass er bei seiner Handhabe, beispielsweise beim Reponieren eines Wirbelkörpers 18 mit einer Hand umgriffen werden kann und die zum Reponieren erforderlichen Kräfte aufgebracht werden können. Beispielsweise ist der Manipulator so bemessen, dass die ersten Wandsegmente 27 eine Länge von etwa 15 cm aufweisen. Die ersten Wandsegmente 27 sind so ausgelegt, dass sie so in gewissem Maße radial aufweitbar bzw. etwa radial voneinander weg bewegt werden können und aufgrund elastischer Rückstellkräfte wieder in die Ausgangslage zurückkehren. Die Greifelemente 28 können daher in einfacher Weise auf den Schraubenkopf 3 aufgesteckt werden. Eine derartige radiale Aufweitbarkeit oder Flexibilität der ersten Wandsegmente 27 ist insbesondere dann vorteilhaft, wenn an den Innenseiten der Greifelemente 28 ein Hintergriffselement 29 radial nach innen vorsteht, welches
eine jeweils an den Wandabschnitten 7 des Schraubenkopfs 3 vorhandene, radial nach innen vorspringende, zum Schraubenschaft 4 weisende Gegenfläche 30 (Fig. 1, 2 und 10) hintergreift. Die Gegenfläche 30 ist beispielsweise eine sich in Umfangsrichtung des Schraubenkopfes 3 erstreckende Wandung einer in den Wandabschnitten 7 des Schraubenkopfes 3 eingebrachten Ausnehmung 34. Durch die genannte Ausgestaltung wird die Festigkeit, mit der ein Schraubenkopf 3 von den Greifelementen 28 gehalten werden kann, insbesondere beim Reponieren eines Wirbelkörpers 18, wesentlich erhöht.

Ein Auseinanderweichen der Greifelemente 28, was zwar beim Aufstecken des Schraubenmanipulators auf einen Schraubenkopf 3 vorteilhaft, nicht jedoch bei der Manipulation der Pedikelschraube etwa während der Reponierung eines Wirbelkörpers 18, wird durch den Schieber 24 verhindert. Dieser ist mit den ersten Wandsegmenten 27 so verbunden, dass er diese zumindest in einer proximalen Verschiebe-Endlage gegen ein radiales Auseinanderweichen sichert. Wie dies bei einer bevorzugten Ausführungsvariante bewerkstelligt wird, wird weiter unten näher erläutert.

Während bei der am offenen Rücken vorgenommenen Wirbelsäulenstabilisierung ein Stab 8 von oben her in die Kopf-Ausnehmungen 13 einer Reihe von Pedikelschrauben 1 eingelegt werden kann, wird bei der perkutanen Operationstechnik der Stab 8 über eine Operationsöffnung in eine Ausnehmung 13 der der Operationsöffnung zugeordneten Pedikelschraube eingeführt und dann so in Längsrichtung der Wirbelsäule weiter geschoben, dass er die Aufnahmen 13 in Stablängsrichtung aufeinander folgender Pedikelschrauben durchsetzt. Wenn ein Stab 8 zu nahe an der Kopfstirnseite positioniert ist, weil er beispielsweise durch Körpergewebe in Distalrichtung gedrückt wird, ist das Einschrauben der Fixierschraube 5 in die Ausnehmung 13 behindert oder gar nicht möglich, weil das Außengewinde 12 der Fixierschraube 5 nicht oder nur unzureichend in das Innengewinde 16 der Pedikelschraube 1 eingreifen kann. Um dieses Problem zu vermeiden, weist der Schieber 24 an seinem proximalen Ende wenigstens ein Niederhalteelement 35 auf, welches in der proximalen Verschiebe-Endlage des Schiebers 24 den in einer Ausnehmung 13 eingeführten Stab 8 innerhalb der Aufnahmenut hält, so dass die Fixierschraube 5 behinderungsfrei in den Schraubenkopf 3 eingeschraubt und der Stab 8 fixiert werden kann.

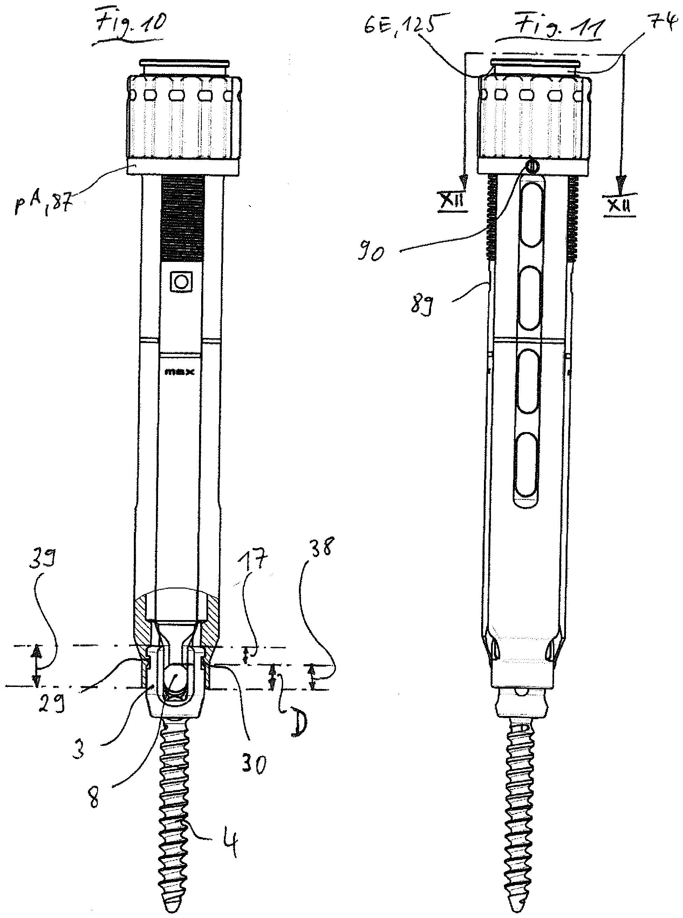

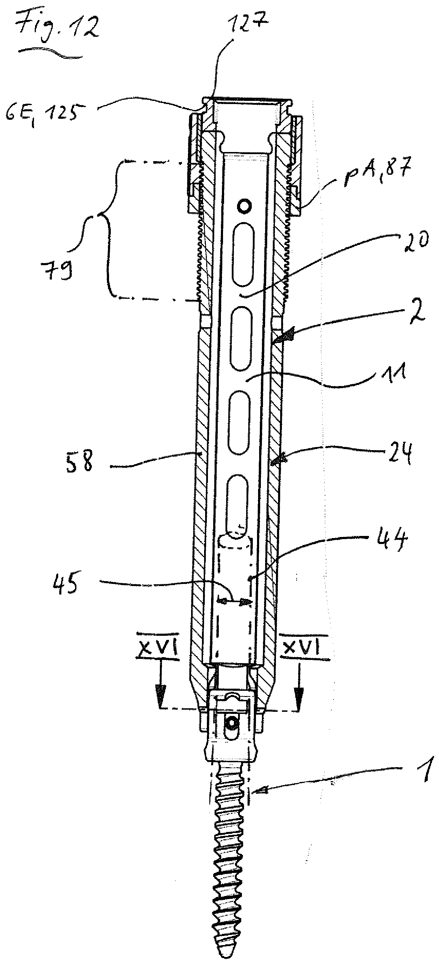

Diese weitere Funktion des Schiebers 24 ist besonders dann gewährleistet, wenn das wenigstens eine Niederhalteelement 35 in der proximalen Schiebe-Endstellung des Schiebers 24 einen Axialabstand 38 zur Stirnseite 25 der Hülse 20 beziehungsweise der ersten Wandsegmente 27 aufweist, der kleiner ist als die Eintauchtiefe 39, mit welcher der Schraubenkopf 3 in einen von den Greifelementen 28 seitlich begrenzten Aufnahmeraum 37 des Schraubenmanipulators 2 axial hineinragt (siehe Fig. 10). Dabei ist es besonders zweckmäßig, wenn der Axialabstand 38 kleiner oder gleich ist der Differenz D aus der Eintauchtiefe 39 des Schraubenkopfs 3 und der Gewindelänge 17 des Innengewindes 16. Die Fixierschraube 5 kann dann zunächst praktisch vollständig in das Innengewinde 16 eingeschraubt werden, bevor gegen Ende des Einschraubvorgangs die Fixierschraube 5 den Stab 8 beaufschlagt und gegen den Grund der Ausnehmung 13 drückt.
Eine Behinderung des Einschraubens der Fixierschraube 5 in den Schraubenkopf 3 mit Hilfe eines in den Hohlraum 11 des Schraubenmanipulators 2 eingesteckten Schraubendrehers (nicht gezeigt) wird dadurch verhindert, dass das wenigstens eine Niederhalteelement 35 außerhalb eines koaxial zur Mittellängsachse 40 der Hülse 20 verlaufenden gedachten Zylinders 44 angeordnet ist, wobei dessen Durchmesser 45 (Fig. 12) dem Kerndurchmesser des Innengewindes 16 des Schraubenkopfes 3 beziehungsweise dem Durchmesser 46 des Außengewindes 12 der Fixierschraube 5 im Wesentlichen entspricht. Wenn das Niederhalteelement 35 noch weiter radial außerhalb, nämlich außerhalb eines gedachten Zylinders 44 angeordnet ist, dessen Durchmesser 45 dem Außendurchmesser 49 des Schraubenkopfs 3 entspricht ist es möglich, eine Fixierschraube einzusetzen, an deren Unterseite eine über deren Außengewinde 12 hinausragende, die Umfangsfläche des Stabes 8 beaufschlagende Druckplatte 50 angeordnet ist. Die Druckplatte 50 ist vorzugsweise so mit der Fixierschraube 5 verbunden, dass sie um deren Mittellängsachse 54 drehbar und außerdem hinsichtlich dieser Achse in jeder Drehstellung verkippbar ist. Auf ihrer proximalen Seite weist die Druckplatte 50 eine komplementär zur Oberflächenkrümmung des Stabes 8 ausgestaltete rinnenförmige Ausnehmung 55 auf. Die Druckplatte 50 steht mit zwei sich diametral gegenüberliegenden Fortsätzen 56 über das Außengewinde 12 der Fixierschraube 5 hinaus, wobei sich die rinnenförmige Ausnehmung in die proximale Seite der Fortsätze 56 hinein erstreckt.

Der Schieber 24 ist ein Hohlzylinder, dessen Wand von zwei diametral gegenüberliegenden, axial verlaufenden und in die proximale Stirnseite des Hohlzylinders ausmündenden Fenstern 57 in zwei zweite Wandsegmente 58 unterteilt ist. Die zweiten Wandsegmente 58 sind in den Fenstern 26 der Hülse 20 axial verschiebbar geführt. Die proximalen Stirnseiten bilden jeweils ein Niederhalteelement 35. In der proximalen Endstellung E nehmen die Niederhalteelemente 35 die oben beschriebene axiale Position gegenüber der Stirnseite 25 der Hülse 20 ein. Die Krümmung der ersten und zweiten Wandsegmente 27, 58 ist so gewählt, dass deren Außenflächen in einer gemeinsamen Zylinderhüllfläche verlaufen, so dass sich insgesamt eine kreisrunde Umfangsform des Schraubenmanipulators 2 ergibt. Außerdem erstrecken sich die ersten Wandsegmente 27 über eine größere Bogenlänge als die zweiten Wandsegmente 58. Diese Abmessungsrelation ist vorteilhaft, weil beispielsweise bei der Reponierung eines Wirbelkörpers 18 relativ große Kräfte in die ersten Wandsegmente 27 beziehungsweise in die von deren Freienden gebildeten Greifelemente 28 eingeleitet werden müssen. Die zweiten Wandsegmente 58 weisen außerdem eine solche Länge auf, dass in der proximalen Verschiebe-Endlage des Schiebers 24 der Schraubenmanipulator 2 insgesamt ein in Umfangsrichtung geschlossener Hohlzylinder ist. Die ersten Wandsegmente 27 können, beispielsweise aus Gründen der Gewichtseinsparung, von Ausnehmungen 59 durchsetzt sein. Gleiches ist auch für die zweiten Wandsegmente 58 denkbar.

Ein radiales Aufweiten der Greifelemente 28 in der proximalen Verschiebe-Endlage E des Schiebers 24 wird bei einer bevorzugten Ausführungsvariante dadurch bewerkstelligt, dass aus den in Umfangsrichtung 60 des Schraubenmanipulators 2 weisenden Seitenkanten der zweiten Wandsegmente 58 eine Rippe 64 vorsteht, welche sich in Axialrichtung erstreckt, wobei in den Seitenkanten der ersten Wandsegmente 27 eine Nut 65 vorhanden ist, in welche die Rippen 64 mit einem in Umfangsrichtung 60 wirksamen Formschluss axial verschiebbar eingreifen. Je weiter sich also der Schieber 24 seiner proximalen Endstellung E nähert, beziehungsweise je kürzer die vom Schieber 24 nicht erfassten Längsabschnitte der ersten Wandsegmente 27 sind, desto geringer ist deren radiale Aufweitbarkeit. In der proximalen Endstellung E ist eine radiale Aufweitbarkeit nicht mehr gegeben, so dass die Greifelemente 28 den Schraubenkopf 3 mit hoher Festigkeit zwischen sich einklemmen beziehungsweise im Aufnahmeraum 37 festhalten.

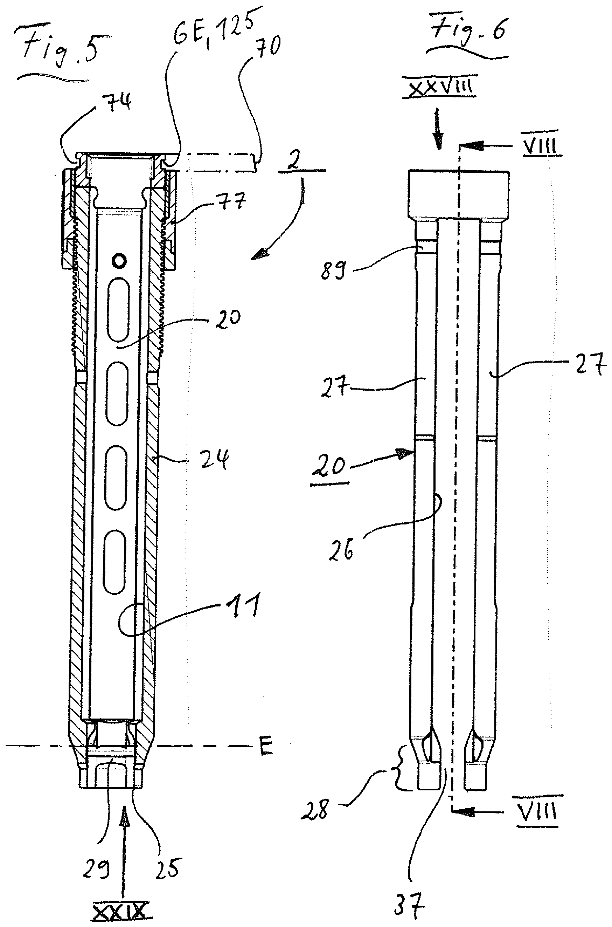

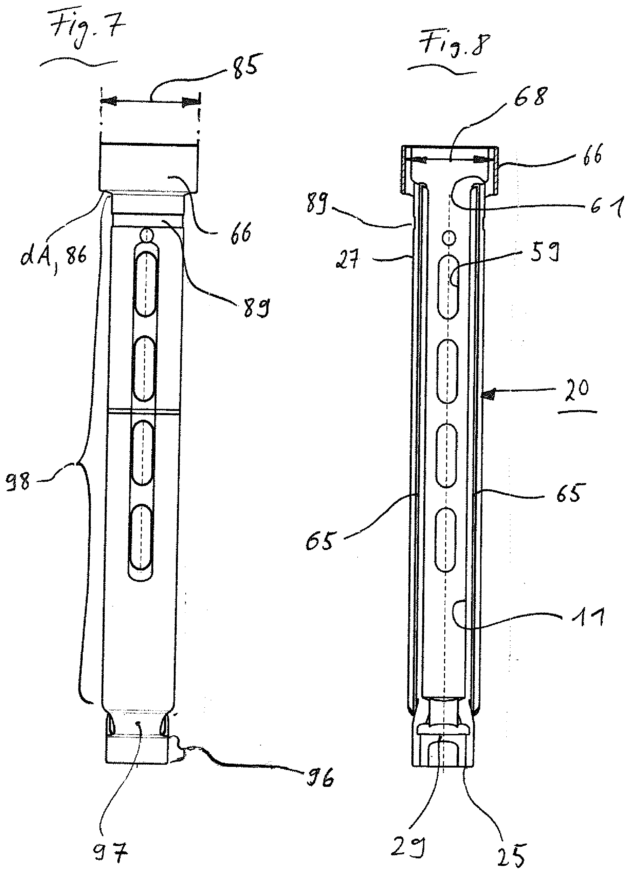

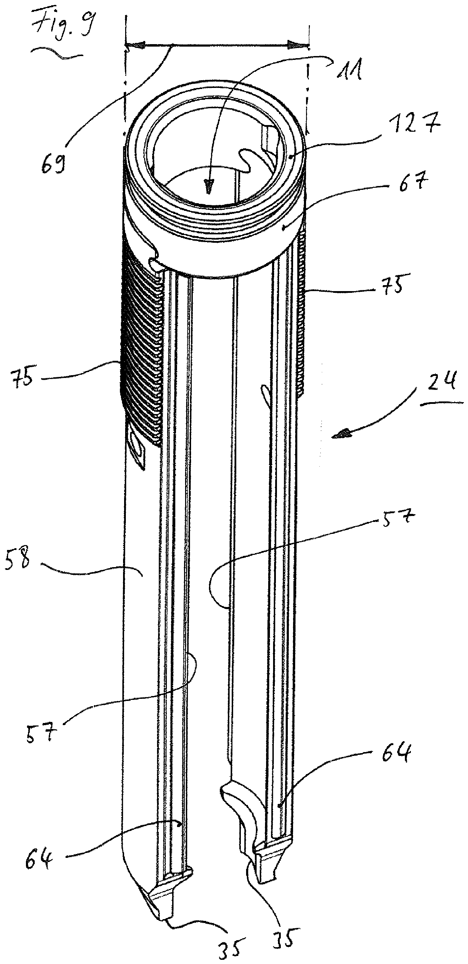

Die distalen Enden der ersten Wandsegmente 27 sind über einen ersten Zylinderabschnitt 66 und die distalen Enden der zweiten Wandelemente 58 über einen zweiten Zylinderabschnitt 67 miteinander verbunden. Die ersten und zweiten Wandelemente 27, 58 sind mit dem jeweiligen Zylinderabschnitt einstückig ausgebildet, wobei sie mit dem proximalen Ende der Zylinderabschnitte verbunden sind. Der Innendurchmesser 68 (Fig. 8) des ersten Zylinderabschnitts 66 ist größer als der Außendurchmesser 69 (Fig. 9)des zweiten Zylinderabschnitts 67. Die Axiallängen der Zylinderabschnitte sind so bemessen, dass der zweite Zylinderabschnitt 67 mit einem Axialüberstand 70 über den ersten Zylinderabschnitt 66 hinaussteht (Fig. 5). In der Umfangsfläche des Axialüberstands 70 ist eine Ringnut 74 vorhanden.

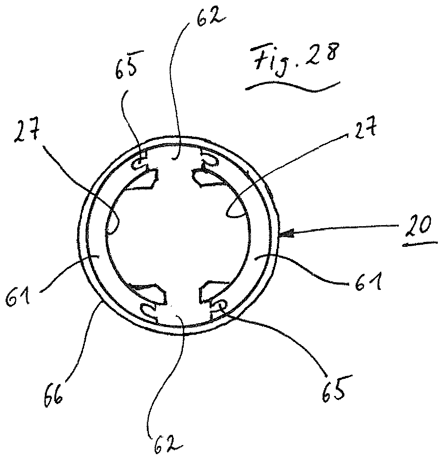

Der Schieber 24 ist von der distalen Stirnseite der Hülse 20 her in diese einsteckbar. Wie insbesondere aus Fig. 8 und Fig. 28 hervorgeht, sind die ersten Wandbereiche 27 der Hülse 20 gegenüber der Außenumfangsfläche des ersten Zylinderabschnitts 66 radial nach innen versetzt, wobei sie mit einer Schulter 61, die vorzugsweise Teil einer sich distal erweiternden Konusfläche ist, in die Innenwandung des ersten Zylinderabschnitts 66 übergeht. In der Verschiebeendlage E steht der Schieber mit der proximalen Stirnseite des zweiten Zylinderabschnitts, die gegebenenfalls komplementär zur Konusform der Schulter 61 ausgebildet ist, an dieser an. Die sich in Umfangsrichtung zwischen den Schultern 61 erstreckenden Zwischenräume 62 nehmen die zweiten Wandsegmente 58 des Schiebers 24 auf.

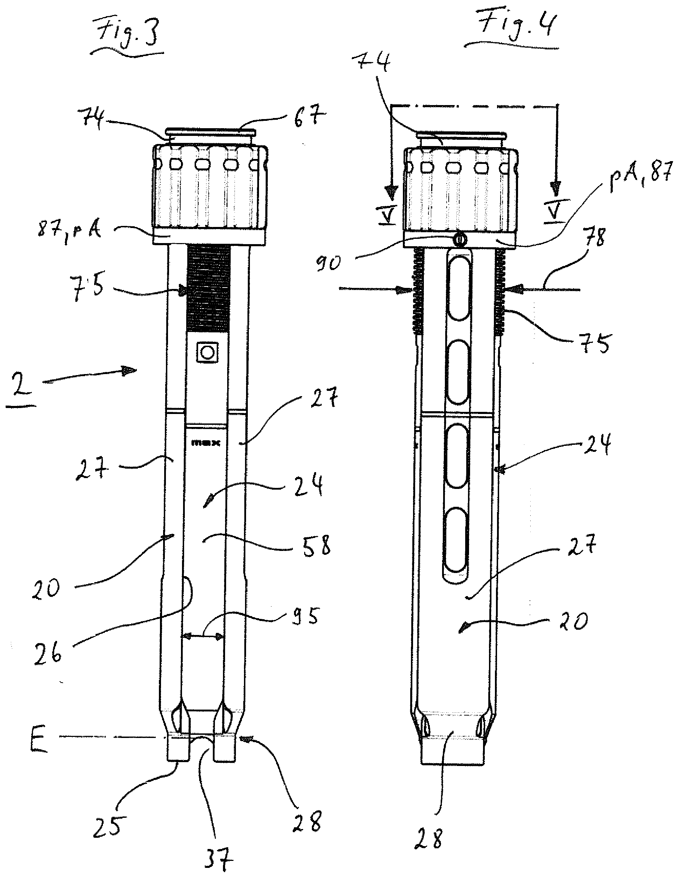

Die axiale Bewegung des Schiebers 24 gegenüber der Hülse 20 erfolgt über ein Schraubgetriebe. Dieses ist dadurch bewerkstelligt, dass aus der Außenfläche der zweiten Wandsegmente 58 des Schiebers 24 ein Außengewinde 75 vorsteht, welches mit einem Innengewinde 76 einer die Hülse 20 koaxial umgreifenden axial fixierten, jedoch drehbaren Verstellmutter 77 zusammenwirkt. Der Außendurchmesser 78 (Fig. 4) des Außengewindes 75 ist kleiner als der Innendurchmesser 68 (Fig. 8) des ersten Zylinderabschnitts 66 der Hülse 20. Aufgrund dieser Abmessungsrelation kann der Schieber 24 behinderungsfrei durch den ersten Zylinderabschnitt 66 hindurch geschoben werden. Das Außengewinde 75 des Schiebers 24 erstreckt sich nicht über die gesamte Länge der zweiten Wandsegmente 58, sondern nur über einen sich an den zweiten Zylinderabschnitt 67 anschließenden Längsabschnitt 79 (Fig. 12). Der Längsabschnitt 79 entspricht etwa einem Viertel der Gesamtlänge der zweiten Wandsegmente 58.

Das Innengewinde 76 erstreckt sich nur über einen Teilbereich der gesamten axialen Länge der Verstellmutter 77. Zwischen dem Innengewinde 76 und der distalen Stirnseite der Verstellmutter 77 ist ein gewindefreier Innenwandbereich 80 vorhanden, wobei dessen Innendurchmesser 84 geringfügig größer ist als der Außendurchmesser 85 des ersten Zylinderabschnitts 66 der Hülse 20. Der Innenwandbereich 80 ist in Axialrichtung so bemessen, dass er den ersten Zylinderabschnitt 66 im Wesentlichen vollständig oder zumindest einen Längsabschnitt davon aufnimmt bzw. umgreift.

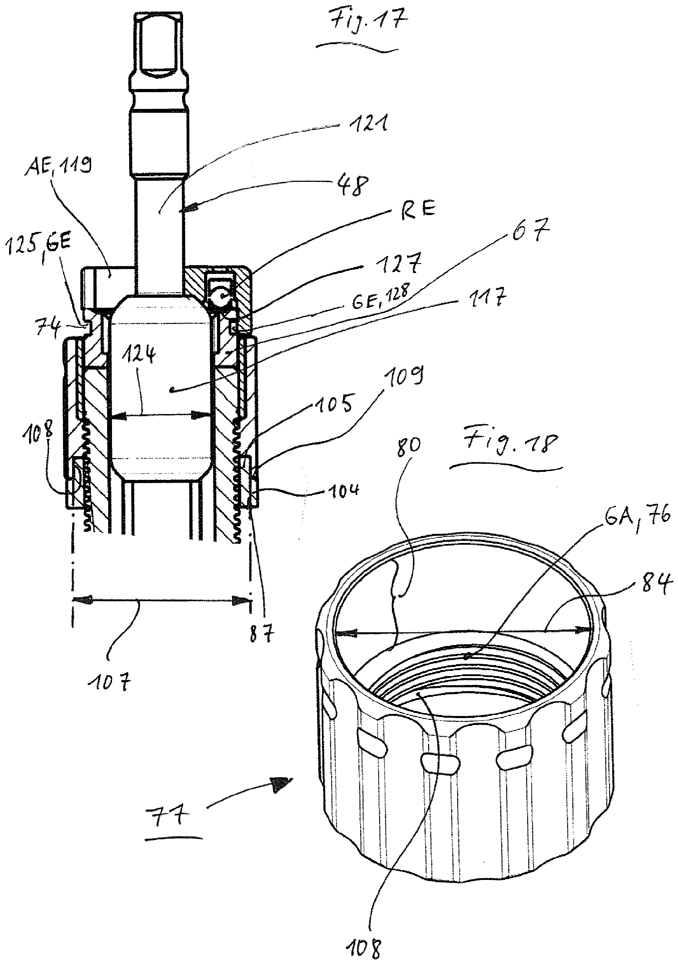

Die axiale Fixierung der Verstellmutter 77 am Schraubenmanipulator 2 wird dadurch bewerkstelligt, dass an den Außenflächen der ersten Wandsegmente 27 mit Axialabstand zueinander ein proximales Anschlagelement pA (z.B. Fig. 3) und ein distales Anschlagelement dA (Fig. 7) vorstehen, welche ein aus der Innenfläche der Verstellmutter 77 radial nach innen ragendes Gegenanschlagelement GA (Fig. 18), welches von dem Innengewinde 76 gebildet ist, zwischen sich einschließen und radial überlappen. Das distale Anschlagelement dA wird von dem ersten Zylinderabschnitt 66 gebildet, wobei dieser mit einer Schulter 86 die Außenflächen der ersten Wandsegmente 27 radial überragt. Das proximale Anschlagelement pA ist ein die Hülse 20 beziehungsweise den Außenumfang des Schraubenmanipulators 2 umgreifender Anschlagring 87, der axialfest mit den ersten Wandsegmenten 27 lösbar verbunden ist. Der Anschlagring 87 greift mit einem in Umfangsrichtung 60 verlaufenden, aus seiner Innenwandung vorstehenden Vorsprung 88 in eine ebenfalls in Umfangsrichtung verlaufende Nut 89 in den Außenflächen der ersten Wandsegmente 27 ein. Eine lösbare Dreh- und Axialfixierung des Anschlagrings 87 erfolgt mit Hilfe einer den Anschlagring 87 radial durchsetzenden Schraube 90, welche im festgezogenen Zustand gegen die Außenfläche der ersten Wandsegmente 27 drückt.

Das proximale, die Greifelemente 28 umfassende Ende der Hülse 20 ist ein radial verengter Längsabschnitt 96, der über einen Konusabschnitt 97 in den sich bis zu dem ersten Zylinderabschnitt 66 erstreckenden Hauptbereich 98 der Hülse 20 übergeht. Die Breite 94 der Vorsprünge 88, deren Innenwandung 99 Teil einer Zylinderfläche ist, ist geringer als die lichte Weite 100 der Fenster 26 im Hauptbereich 98 der Hülse 20. Bei der Montage des Schraubenmanipulators 2, kann daher der Anschlagring 87 auf den Hauptbereich 98 aufgeschoben werden, wenn er sich in einer Drehlage befindet, in der sich seine Vorsprünge 88 in dem von den Fenstern eingenommenen Umfangsabschnitt der Hülse 20 befinden. Die Vorsprünge 88 erstrecken sich in Axialrichtung von der distalen Stirnseite des Anschlagrings 87 weg, jedoch nur über eine Teillänge des Anschlagrings 87. Ein sich proximal von einem der beiden Vorsprünge 88 weg erstreckender Innenwandbereich ist von der Schraube 90 durchgriffen.

Der Anschlagring 87 weist einen proximalen Längsabschnitt 104 und einen gegenüber diesem radial verengten Längsabschnitt 105 auf. Dessen Außendurchmesser 106 ist geringfügig kleiner als der Innendurchmesser 107 eines sich proximal an das Innengewinde 76 anschließenden Innenwandbereichs 108 der Verstellmutter 77 (Fig.17, 18). Die Axiallänge des verengten Längsabschnitts 105 ist so gewählt, dass dieser vollständig in den Innenwandbereich 108 der Verstellmutter 77 eintaucht. Die Verstellmutter 77 liegt dabei mit ihrer proximalen Stirnseite auf einer Radialschulter 109 auf, welche den Übergang zwischen den Längsabschnitten 104 und 105 bildet.

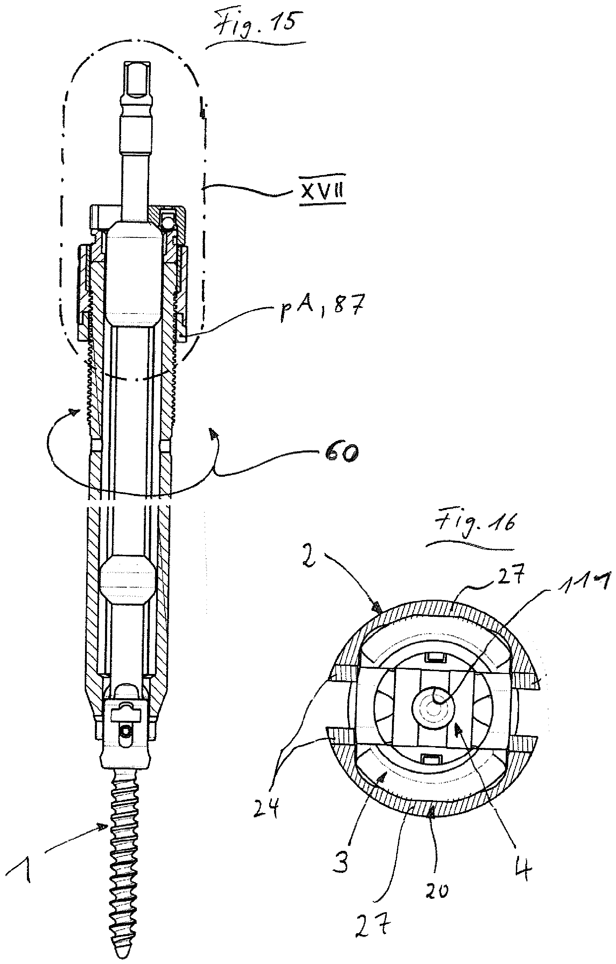

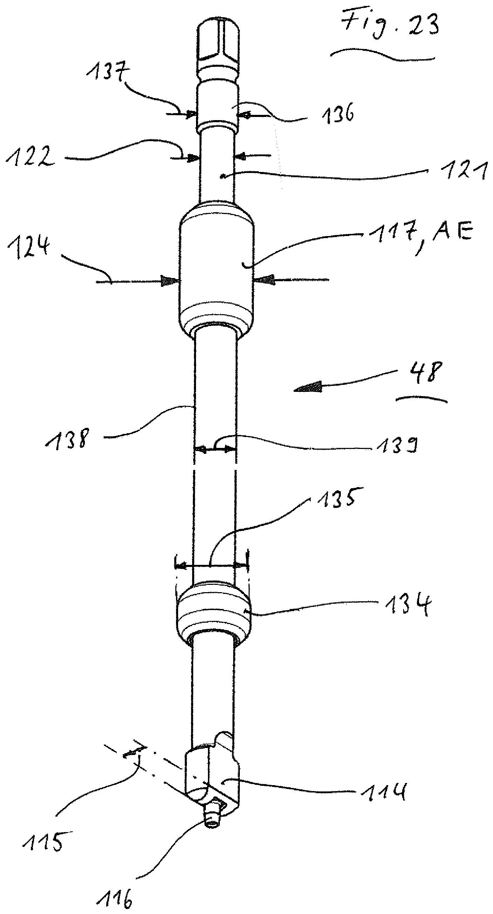

Der Instrumentensatz umfasst weiterhin den oben schon erwähnten Schraubendreher 48. Dieser ist stabförmig ausgebildet und ist in den zentralen Hohlraum 19 des Schraubenmanipulators 2 einführbar bzw. im Anwendungsfall darin eingeführt. Am proximalen Ende des Schraubendrehers ist ein drehfest in die Ausnehmung 13 des Schraubenkopfes 3 einsteckbares Drehbetätigungselement 114 angeordnet. Dieses weist etwa die Form einer Platte auf, deren Breite 115 geringfügig kleiner ist als die Breite 9 der Zwischenräume 16 des Schraubenkopfs 3. Optional steht aus der proximalen Stirnseite des Schraubendrehers 48 bzw. des Drehbetätigungselements 114 ein Zapfen 116 axial vor, welcher vorgesehen ist, um in eine Bohrung 111 (Fig. 16) im distalen Ende des Schraubenschafts 4 eingesteckt zu werden und dabei den Schraubenschaft in einer koaxialen Ausrichtung bezüglich der Mittellängsachse 10 des Schraubenkopfes 3 zu fixieren.
Damit der Schraubendreher 48 zuverlässig in der Ausnehmung 13 des Schraubenkopfes 3 gehalten wird bzw. mit ausreichender Eintauchtiefe in die Ausnehmung 13 hinein ragt, ist eine Arretiereinrichtung AE (siehe Fig. 23 ff.) vorgesehen, mit welcher der in den Hohlraum 11 des Schraubenmanipulators 2 eingeführte Schraubendreher 48 in einer entsprechenden axialen Sollposition gehalten wird. Zu diesem Zweck umfasst die Arretiereinrichtung AE vorzugsweise einen aus der Umfangsfläche des Schraubendrehers 48 vorstehenden Vorsprung 117 und ein diesen in der Sollposition des Schraubendrehers 48 auf seiner Distalseite radial überlappendes, gegenüber dem Schraubenmanipulator axialfestes Arretierelement, welches im Wesentlichen als Scheibe 119 ausgebildet ist. Die axiale Position des Vorsprungs 117 am Schraubendreher 48 ist dabei so gewählt, dass die arretierende Radialüberlappung des Vorsprungs 117 durch das Arretierelement nur dann herstellbar ist, wenn sich der Schraubendreher 48 in seiner Sollposition befindet.

Der Vorsprung 117 erstreckt sich über den gesamten Umfang des stabförmigen Schraubendrehers 48, ist also als Ringvorsprung ausgebildet. Aufgrund dieser Ausgestaltung ist das Zusammenwirken mit dem Arretierelement unabhängig von der jeweiligen Drehstellung des Schraubendrehers 48 bezüglich seiner Mittellängsachse möglich. Vorzugsweise ist das Arretierelement AE eine von einer Radialnut 118 durchsetzte Scheibe 119, insbesondere eine Kreisscheibe. Die Radialnut 118 öffnet sich in den Rand der Scheibe 119, so dass diese von der Seite her bzw. in Radialrichtung auf den Schraubendreher 48 aufgesteckt werden kann. Sie erstreckt sich außerdem so weit radial nach innen, dass die Scheibe 119 vom Schraubendreher zentral durchgriffen ist. Die lichte Weite 120 der Radialnut 118 ist größer als die Dicke oder der Durchmesser 122 eines sich distal vom Vorsprung 117 weg erstreckenden Längsabschnitts 121 des Schraubendrehers 48. Die lichte Weite 120 ist aber kleiner als die Abmessung des Vorsprungs 117 in einer sich quer zur Mittellängsachse des Schraubendrehers 48 erstreckenden Richtung. Im Falle eines ringförmig ausgebildeten Vorsprungs 117 ist somit die lichte Weite 120 der Radialnut 118 kleiner als der Durchmesser 124 des Vorsprungs 117. Eine lösbare axialfeste Verbindung zwischen Schraubenmanipulator 2 und der Scheibe 119 erfolgt über eine in Radialrichtung, also einer sich rechtwinklig zur Mittellängsachse 40 des Schraubendrehers 48 erstreckenden Richtung, wirksame Rastverbindung zwischen der Scheibe 119 und dem Schraubenmanipulator 2. Zu diesem Zwecke steht aus der proximalen Seite der Scheibe 119 ein in Rastrichtung federnd beaufschlagtes Rastelement RE hervor, das beispielsweise als Kugel ausgebildet ist. Das Rastelement RE ist in einer in die proximale Seite der Scheibe 119 mündenden Bohrung 130 gehalten, wobei es aus dieser herausragt, ausgebildet ist. Das Rastelement RE greift in eine Ringnut 127 (Fig. 9, 17) in der distalen Stirnseite des Schiebers 24 ein.

Auf der proximalen Seite der Scheibe 119 ist ein Hintergriffselement HE angeordnet, welches zur axialen Arretierung des Schraubendrehers 48 am Schraubenmanipulators 2 mit einer sich quer zu dessen Mittellängsachse 40 erstreckenden Fügerichtung auf den Schraubenmanipulator 2 aufsteckbar ist, wobei es im aufgesteckten Zustand ein am Schraubenmanipulator 2 angeordnetes Gegenelement GE hintergreift. Das Gegenelement GE ist die zum proximalen Ende des Schraubenmanipulators 2 weisende Nutwand 125 der im zweiten Zylinderabschnitt 67 vorhandenen Ringnut 74. Das Hintergriffselement HE ist beispielsweise ein mit Axialabstand zur proximalen Seite der Scheibe 119 angeordneter u-förmig verlaufender Flansch 128. Dieser öffnet sich in die gleiche Richtung wie die Radialnut 118 und ist an einer u-förmigen Schürze 129 angeformt, welche sich von der proximalen Seite der Scheibe 117 wegerstreckt. Der Vorsprung 117 hat neben seiner Arretierfunktion auch noch die Aufgabe, den Schraubendreher kippfest im Innenraum 11 des Schraubenmanipulators 2 zu halten. Seine Abmessung in einer quer zur Mittellängsachse 40 des Schraubendrehers 48 verlaufenden Richtung sind, um dazu so gewählt, dass zwischen dem Vorsprung 117 und der Innenwand des Innenraums 11 kein oder nur ein geringes Spiel vorhanden ist. Ebenfalls zu dem genannten Zweck ist am Schraubendreher 48 ein weiterer Vorsprung 134 angeformt und zwar mit Axialabstand in Proximalrichtung zum Vorsprung 117. Der Vorsprung 134 ist beispielsweise als Ringvorsprung ausgebildet, wobei sein Durchmesser 135 dem Durchmesser 124 des Vorsprungs 117 entspricht.

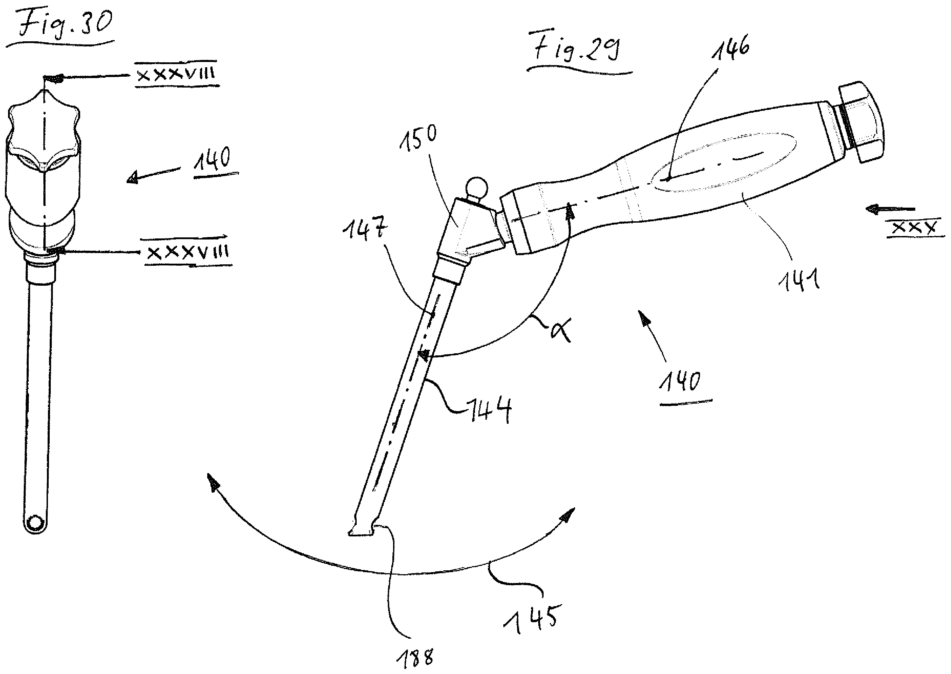

Der Schraubendreher 48 weist schließlich noch einen weiteren, beispielsweise ringförmigen Vorsprung 136 auf, der mit Axialabstand zum Vorsprung 117 am Längsabschnitt 121 angeordnet ist. Die Abmessung des Vorsprungs 136 in einer sich quer zur Mittellängsachse des Schraubendrehers 48 erstreckenden Richtung bzw. sein Durchmesser 137 ist kleiner als der Durchmesser 124 des Vorsprungs 117 jedoch größer als die lichte Weite 120 der Radialnut 118 des Arretierelements AE bzw. der Scheibe 119. Der sich zwischen den Vorsprüngen 117 und 134 erstreckende Längsabschnitt 138 hat einen Durchmesser 139, der größer ist als die lichte Weite 120 der Radialnut 118. Das Arretierelement AE bzw. die Scheibe 119 kann somit nicht versehentlich auf den sich vom Vorsprung 117 proximal weg erstreckenden Längsabschnitt 138 aufgesteckt werden.
Ein weiterer optionaler Bestandteil des Instrumentensatzes ist ein zur Manipulation eines Stabes 8 dienendes Greifwerkzeug 140, welches auch unabhängig von dem oben beschriebenen Schraubenmanipulator 2 eingesetzt werden kann. Das Greifwerkzeug 140 weist einen stabförmigen Schaft 144 auf, an dessen proximalem Ende ein zum festhalten eines Stabes 8 dienendes Greifelement und an dessen anderem Ende ein lang gestreckter Handgriff 145 angeordnet ist. Der Handgriff 145 ist so ausgerichtet, dass seine Längsrichtung 146 mit der Längsrichtung 147 des Schaftes 144 einen Winkel α < 180° und > 90° einschließt. Diese Ausgestaltung ist in ergonomischer Hinsicht gegenüber solchen Greifwerkzeugen für den genannten Zweck verbessert, bei denen sich der Handgriff im Wesentlichen koaxial zum Schaft 144 erstreckt. Das Greifwerkzeug 114 erlaubt eine bequemere Handhaltung beim Einführen eines Stabes in die Ausnehmungen 13 von der in der Wirbelsäule implantierten Pedikelschrauben 1. Insbesondere ist ein Verschwenken des Schaftes 144 etwa in Richtung des Doppelpfeils 145 in Fig. 30 einfacher und mit entspannter Handhaltung durchführbar. Ein solches Verschwenken des Schaftes 144 kommt etwa dann in Frage, wenn nicht ein geradlinig verlaufender, sondern ein gebogener Stab 8 über eine punktuelle Hautöffnung in das zu stabilisierende Gebiet der Wirbelsäule eingeführt werden soll. Vorzugsweise ist der Winkel zwischen dem Handgriff 141 und dem Schaft 144 so gewählt, dass der Winkel α in einem Bereich von 100° bis 140°, besser von 110° bis 130° liegt. Bei dem in Fig. 30 ff. dargestellten Ausführungsbeispiel liegt ein Winkel α von etwa 120° vor.

Der Schaft 144 und der Handgriff 141 sind jeweils in Längsrichtung 146, 147 von einem Hohlraum 148, 149 durchsetzt. Die Hohlräume sind an der Verbindungsstelle 150 von Handgriff und Schaft miteinander verbunden und nehmen eine Betätigungseinrichtung 154 auf, welche dazu vorgesehen ist, ein Greifelement des Werkezuges in eine einen Stab einklemmende Greifstellung und in eine den Stab frei gebenden Freigabestellung zu überführen. Die Betätigungseinrichtung 154 umfasst ein axial beweglich im Hohlraum 148 des Handgriffes 141 angeordnetes stabförmiges Antriebselement 155 und ein axial beweglich im Hohlraum 149 des Schaftes 144 angeordnetes stabförmiges Abtriebselement 156. An der Verbindungsstelle 150 bilden die dort einander zugewandten Enden der genannten Elemente ein Keilgetriebe. Dabei ist besonders vorteilhaft, dass außer dem Antriebselement 155 und dem Abtriebselement 156 keine weiteren Bauteile erforderlich sind, um eine zur Überführung des Greifelementes in seine Greif- oder Freigabestellung erforderliche längsaxiale Bewegung des Antriebselementes 155 in eine längsaxiale Bewegung des Abtriebselements 156 umzuwandeln. Für eine möglichst effektive Kraftübertragung ist es dabei zweckmäßig, wenn eine am Antriebselement 155 oder am Abtriebselement 156 vorhandene Schrägfläche 157 so ausgerichtet ist, dass sie mit der Längsrichtung 146, 147 des jeweiligen Antriebs- bzw. Abtriebselements einen Winkel β einschließt, welcher mit einer Abweichung von 20° dem halben von den Längsrichtungen 146, 147 eingeschlossenen Winkel α entspricht. Der Wert des Winkels β liegt somit in einem Bereich von 50° bis 70°.

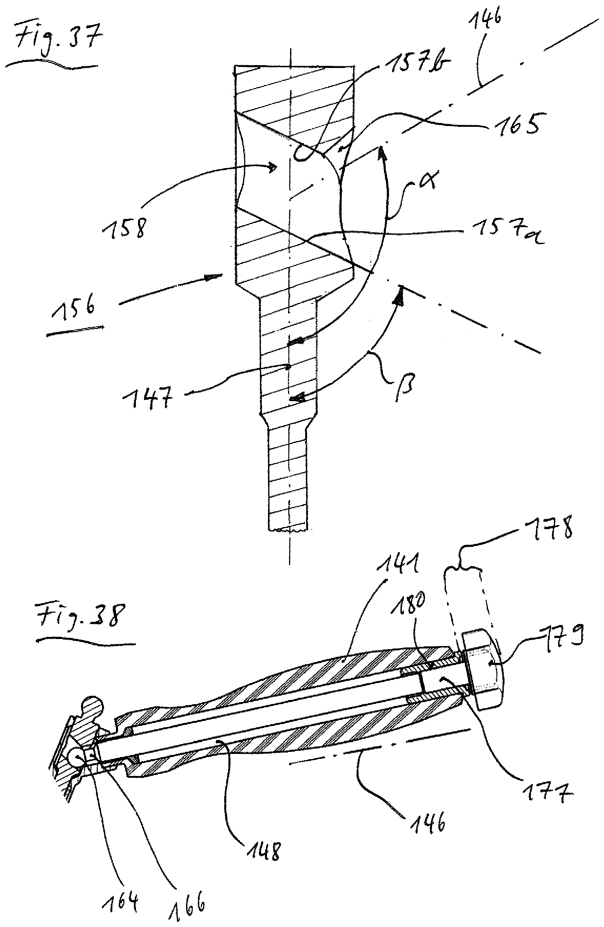

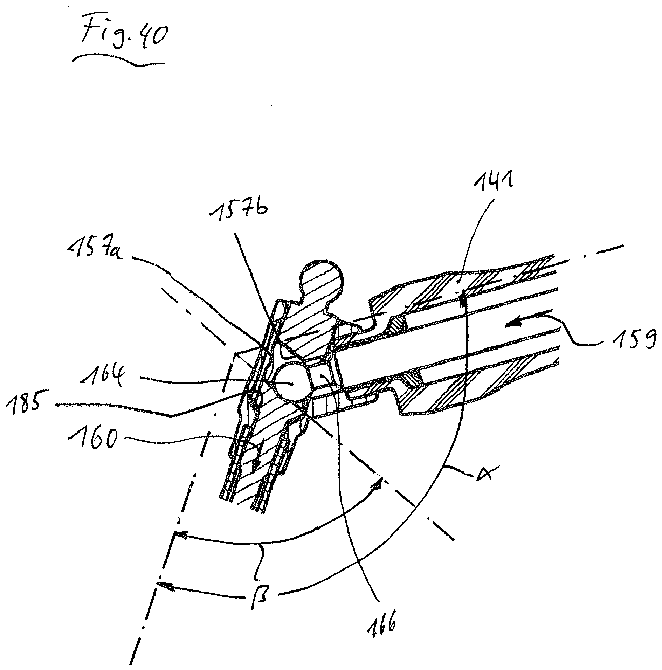

Bei dem dargestellten Ausführungsbeispiel ist das Abtriebselement 156 von einer Bohrung 158 durchsetzt, wobei deren Bohrungswand eine erste und eine zweite Schrägfläche 157a, 157b bildet (Fig.38). Damit sich die Ausrichtung der Bohrung 158 und der Schrägflächen 157 nicht verändert, ist das Abtriebselement 156 drehfixiert im Hohlraum 149 gelagert. Die erste Schrägfläche 157a weist etwa in Distalrichtung und wird dann vom Antriebselement 155 beaufschlagt, wenn dieses in Richtung zum Schaft 144 bewegt wird (Pfeil 159 in Fig. 40). Dabei wird das Abtriebselement 156 zum Freiende des Schafts 144 hin bewegt, was durch den Pfeil 160 in Fig. 41 angedeutet ist. Die zweite Schrägfläche 157b, welche etwa in Proximalrichtung weist und die gleiche Ausrichtung aufweist wie die erste Schrägfläche 157a wird von dem Antriebselement 155 beaufschlagt, wenn dieses in umgekehrter Richtung (entgegen Pfeil 159) bewegt wird. Als Folge davon wird das Abtriebselement 156 in Richtung zur Verbindungsstelle 150 (entgegen Pfeil 160) bewegt. Mit der Bewegung des Abtriebselements in die eine oder andere Richtung kann ein Greifelement gesteuert, d.h. in seine Greif- oder Freigabestellung bewegt werden, was für das vorliegende Ausführungsbeispiel weiter unten näher erläutert wird.

Vorzugsweise hat die Bohrung 158 einen kreisrunden Bohrungsquerschnitt. Durch diese Ausgestaltung ergibt sich die Möglichkeit, ein Antriebselement 155 zu verwenden, das drehbar und axial verschiebbar im Handgriff gelagert ist, so dass der Vorschub in Richtung des Pfeils 159 und der Rückschub entgegen dem Pfeil 159 durch einen Schraubenantrieb erfolgen bzw. das Antriebselement 155 als Schraube ausgebildet sein kann. Dabei ist es vorteilhaft, das mit einer Schrägfläche 157a, 157b zusammenwirkende Ende des Antriebselements 155 oder in kinematischer Umkehr das Ende des Abtriebselements 156 als Kugel 164 auszubilden. In dem dargestellten Ausführungsbeispiel ragt das die Kugel 164 tragende Ende des Antriebselements 155 in die Bohrung 158 hinein. Der Durchmesser der Kugel 164 ist so bemessen, dass sie in der Bohrung 158 spielfrei oder mit geringem Radialspiel geführt ist. Um angesichts der gewinkelten Anordnung von An- und Abtriebselement eine Kollision des sich in die Bohrung 158 hinein erstreckenden Endes des Antriebselementes 155 mit dem dem Handgriff 141 zugewandten Bohrungsrand zu verhindern, weist dieser in einem distalen Bereich eine Ausnehmung 165 auf. Außerdem ist die Kugel 164 über einen radial verengten Hals 166 mit dem restlichen Längsabschnitt des Antriebselementes 155 verbunden.

Das Antriebselement 155 ist als Schraube ausgebildet, weist also ein Außengewinde 177 auf, das mit einem Innengewinde 180, welches an der Innenwandung des Hohlraums 148 angeordnet ist, zusammen wirkt. Weiterhin ragt das Antriebselement 155 mit einem Überstand 178 aus dem dem Schaft 144 abgewandten Ende des Handgriffes 144 heraus, wobei an dem Überstand 178 ein zur Drehbetätigung des Antriebselementes 155 dienendes Handrad 179 angeordnet ist.

Das in dem Bereich der Verbindungsstelle 150 hinein ragende Ende des Abtriebselements 156, welches von der Bohrung 158 durchsetzt ist, ist radial verbreitert und geht mit einer sich in Proximalrichtung konisch verjüngenden Schulter 184 in einen radial verengten Längsabschnitt über. Der Hohlraum 149 ist im Bereich der Verdichtungsstelle 150 komplementär zu dem oben beschriebenen Ende des Abtriebselements 156 ausgebildet, weist also eine in Distalrichtung aufweisende und sich in dieser Richtung konisch erweiternde Schulter 185 auf, welche einen Anschlag für die Schulter 184 bildet und die Bewegung des Abtriebselements 156 in Proximalrichtung bzw. in Richtung des Pfeils 160 begrenzt.

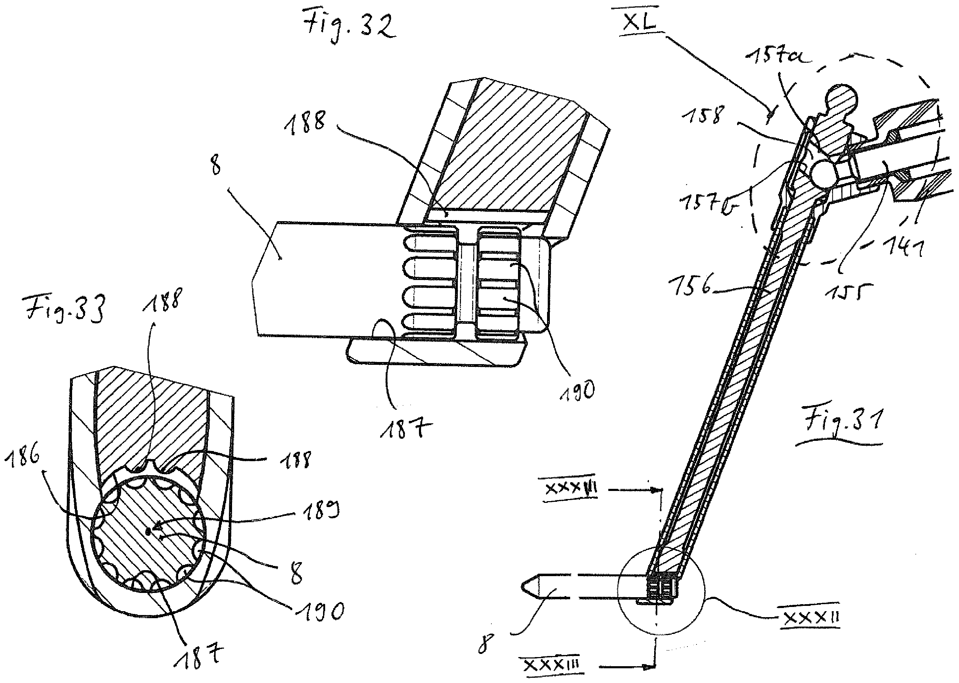

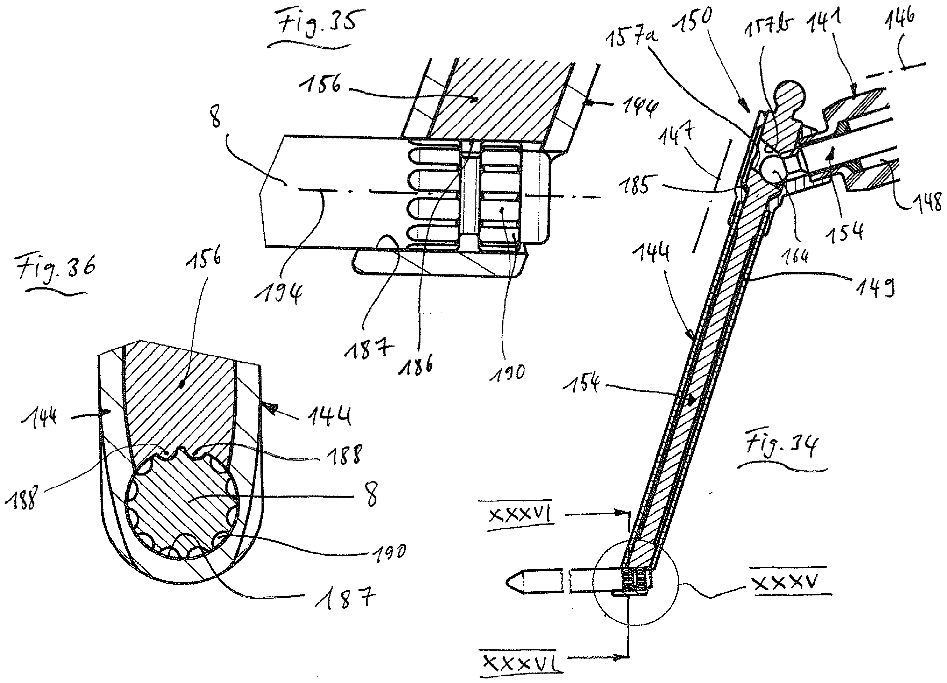

Das oben erwähnte Greifelement wird beim dem vorliegenden Ausführungsbeispiel durch die proximale Stirnseite 186 des Abtriebselements 156 gebildet. Der Schaft 144 ist an seinem Freiende von einer zur Aufnahme des Stabes 8 dienenden, mit dem Hohlraum 149 verbundenen Bohrung 187 durchsetzt. Die Mittellängsachse 195 der Bohrung 188 erstreckt sich in der von den Längsrichtungen 146, 147 des Handgriffes 141 und des Schaftes 144 aufgespannten Ebene. In der Greifstellung (Fig. 34 bis 36) drückt das Greifelement bzw. die Stirnseite 186 den Stab 8 gegen einen in Distalrichtung weisenden Bereich der Bohrungswandung. In der Freigabestellung ist die Stirnseite 186 mit Axialabstand zum Stab 8 angeordnet (Fig. 31 bis 33), so dass der Stab 8 in die Bohrung 187 eingeführt oder aus dieser heraus genommen werden kann. Die Stirnseite 186 ist konkav ausgebildet und ist Teil einer Zylinderfläche, wobei deren Oberflächenkrümmung komplementär zur Krümmung des Stabes 8 ausgebildet ist. Auf diese Weise wird der Stab 8 mit einem Formschluss in der Bohrung 187 gehalten. Um eine drehfeste Fixierung des Stabes 8 in der Bohrung 187 zu bewerkstelligen, steht aus der proximalen Stirnseite 186 wenigstens ein Vorsprung 188 vor. Um ein Drehfixierung des Stabes 8 zu erreichen, weist dieser eine zum wenigstens einen Vorsprung 188 komplementäre Ausnehmung auf, in welche der Vorsprung 188 eingreifen kann. Bei dem vorliegenden Ausführungsbeispiel sind zwei rippenförmige, sich in Richtung der Bohrungsachse 189 erstreckende rippenförmige Vorsprünge 188 an der Stirnseite 186 vorhanden. Ein von der Bohrung 188 aufgenommenes Ende eines Stabes 8 weist vorzugsweise eine Vielzahl von Ausnehmungen 190 auf, welche komplementär zu den rippenförmigen Vorsprüngen 188 ausgebildet. Sie erstrecken sich in Richtung der Mittellängsachse 194 des in die Bohrung 188 eingeführten Stabendes und sind in Umfangsrichtung des Stabes gleichmäßig verteilt. Die Ausnehmungen bilden auf dem Stab 8 eine etwa zahnradähnliche Struktur. Aufgrund der in Rede stehenden Ausgestaltung des Stabs 8 kann dieser in beliebiger Drehlage in die Bohrung 187 eingeführt und darin drehfixiert gehalten werden.

## Patentansprüche

1. Instrumentensatz für die perkutane Wirbelsäulenstabilisierung mit Hilfe von Stäben (8) und einen Schraubenkopf (3) und einen Schraubenschaft (4) aufweisenden Pedikelschrauben (1) mit einer distal offenen Ausnehmung (13) zur Aufnahme eines Stabs (8), mit einem von einem zentralen Hohlraum (11) axial durchsetzten, eine zylindrische Hülse (20) und einen Schieber (24) umfassenden Schraubenmanipulator (2), wobei
- die Wand der zylindrischen Hülse (20) von zwei diametral gegenüberliegenden, axial verlaufenden und in die proximale Stirnseite der Hülse (20) ausmündenden Fenstern (26) in zwei erste Wandsegmente (27) unterteilt ist,
- die proximalen Enden der ersten Wandsegmente (27) bilden Greifelemente (28), welche bei einer Schraubenmanipulation zumindest einen Längsabschnitt des Schraubenkopfes (3) zwischen sich aufnehmen, und
- der Schieber (24) in Axialrichtung an der Hülse (20) verschiebbar und so mit den ersten Wandsegmenten (27) verbunden ist, dass er diese zumindest in einer proximalen Verschiebe-Endlage (E) gegen ein radiales Auseinanderweichen sichert,
- der Schieber (24) ein Hohlzylinder ist, dessen Wand von zwei diametral gegenüberliegenden, axial verlaufenden, in die proximale Stirnseite des Hohlzylinders ausmündenden Fenstern (57) in zwei zweite Wandsegmente (58) unterteilt ist, welche in den Fenstern (26) der Hülse (20) axial verschiebbar geführt sind und deren proximale Stirnseiten jeweils ein Niederhalteelement (35) bilden, **dadurch gekennzeichnet, dass**
- ein erstes oder zweites Wandsegment (27, 58) eine aus seiner in Umfangsrichtung (60) des Schraubenmanipulators (2) weisenden Seitenkante vorstehende Rippe (64) aufweist, welche sich in Axialrichtung erstreckt, und in den Seitenkanten des jeweils anderen Wandsegments (27, 58) jeweils eine sich in Axialrichtung erstreckende Nut (65) vorhanden ist, in welche die Rippe (64) mit einem in Umfangsrichtung wirksamen Formschluss axial verschiebbar eingreift.

2. Instrumentensatz nach Anspruch 1, **dadurch gekennzeichnet, dass** der Schieber (24) an seinem proximalen Ende wenigstens ein Niederhalteelement (35) aufweist, welches in der proximalen Endlage des Schiebers einen in eine distal offene Ausnehmung (13) des Schraubenkopfes (3) eingeführten Stab (8) innerhalb der Ausnehmung (13) hält.

3. Instrumentensatz nach Anspruch 2, **dadurch gekennzeichnet, dass** das wenigstens eine Niederhalteelement (35) in der proximalen Schiebe-Endstellung (E) des Schiebers (24) einen Axialabstand (38) zur Stirnseite (25) der Hülse (20) bzw. der ersten Wandsegmente (27) aufweist, der kleiner ist als die Eintauchtiefe (39), mit der der Schraubenkopf (3) in einen von den Greifelementen (28) seitlich begrenzten Aufnahmeraum (37) axial hineinragt.

4. Instrumentensatz nach Anspruch 3, **dadurch gekennzeichnet, dass** der Axialabstand (38) kleiner oder gleich ist der Differenz aus der Eintauchtiefe (39) und der Gewindelänge (17) eines im Schraubenkopf (3) vorhandenen, zur Fixierung des Stabs (8) mit Hilfe einer Fixierschraube (5) dienenden Innengewindes (16).

5. Instrumentensatz nach Anspruch 2, 3 oder 4, **dadurch gekennzeichnet, dass** das wenigstens eine Niederhalteelement (35) außerhalb eines koaxial zur Mittellängsachse (40) der Hülse (20) verlaufenden gedachten Zylinders (44) angeordnet ist, dessen Durchmesser (45) dem Durchmesser (46) des Außengewindes (12) der Fixierschraube (5) entspricht.

6. Instrumentensatz nach Anspruch 5, **dadurch gekennzeichnet, dass** der Durchmesser (45) des gedachten Zylinders (44) dem Außendurchmesser (49) des Schraubenkopfs (3) entspricht.

7. Instrumentensatz nach einem der vorhergehenden Ansprüche, **dadurch gekennzeichnet, dass** sich die ersten Wandsegmente (27) über eine größere Bogenlänge erstrecken als die zweiten Wandsegmente (58).

8. Instrumentensatz nach einem der vorhergehenden Ansprüche, **dadurch gekennzeichnet, dass** die Außenflächen der ersten und zweiten Wandsegmente (27, 58) in einer gemeinsamen Zylinderhüllfläche verlaufen.

9. Instrumentensatz nach einem der vorhergehenden Ansprüche, **dadurch gekennzeichnet, dass** die distalen Enden der ersten Wandsegmente (27) über einen ersten Zylinderabschnitt (66) und die distalen Enden der zweiten Wandelemente (58) über einen zweiten Zylinderabschnitt (67) miteinander verbunden sind, wobei der Innendurchmesser (68) des ersten Zylinderabschnitts (66) größer ist als der Außendurchmesser (69) des zweiten Zylinderabschnitts (67).

10. Instrumentensatz nach einem der vorhergehenden Ansprüche, **dadurch gekennzeichnet, dass** eine axiale Bewegung des Schiebers (24) gegenüber der Hülse (20) über ein Schraubgetriebe erfolgt.

11. Instrumentensatz nach Anspruch 10 in Verbindung mit Anspruch 7, **dadurch gekennzeichnet, dass** aus der Außenfläche der zweiten Wandsegmente (58) ein Außengewinde (75) vorsteht, welches mit einem Innengewinde (76) einer die Hülse (20) koaxial umgreifenden, axialfixierten Verstellmutter (77) zusammenwirkt.

12. Instrumentensatz nach Anspruch 11, **dadurch gekennzeichnet, dass** zur Axialfixierung der Verstellmutter (77) aus den Außenflächen der ersten Wandsegmente (27) mit Axialabstand zueinander ein proximales und ein distales Anschlagelement (pA, dA) vorstehen, welche ein aus der Innenfläche der Verstellmutter (77) radial nach innen vorstehendes Gegen-Anschlagelement (GA) zwischen sich einschließen und radial überlappen.

13. Instrumentensatz nach Anspruch 12, **dadurch gekennzeichnet, dass** das distale Anschlagelement (dA) von dem ersten Zylinderabschnitt (66) gebildet ist, wobei dieser mit einer Schulter (86) die Außenflächen der ersten Wandsegmente (27) radial überragt.

14. Instrumentensatz nach Anspruch 12 oder 13, **dadurch gekennzeichnet, dass** das proximale Anschlagelement (pA) ein die Hülse (20) umgreifender Anschlagring (87) ist, der axialfest mit den ersten Wandsegmenten (27) lösbar verbunden ist.

15. Instrumentensatz nach einem der Ansprüche 12 bis 14, **dadurch gekennzeichnet, dass** das Gegen-Anschlagelement (GA) das Innengewinde (76) der Verstellmutter (77) ist.

16. Instrumentensatz nach einem der Ansprüche 12 bis 15, **dadurch gekennzeichnet, dass** die Verstellmutter (77) am distalen Ende der Hülse (20) angeordnet ist.

17. Instrumentensatz nach Anspruch 16, **dadurch gekennzeichnet, dass** die Verstellmutter (77) zumindest einen Längsabschnitt des ersten Zylinderabschnitts (66) umgreift.

18. Instrumentensatz nach einem der vorhergehenden Ansprüche, **dadurch gekennzeichnet, dass** er einen stabförmigen, in den zentralen Hohlraum (11) des Schraubenmanipulators (2) einführbaren Schraubendreher (48) umfasst, an dessen proximalem Ende ein in die Ausnehmung (13) des Schraubenkopfes (3) drehfest einsteckbares Drehbetätigungselement (114) angeordnet ist.

19. Instrumentensatz nach Anspruch 18, **gekennzeichnet durch** eine Arretiereinrichtung, mit welcher der in den Hohlraum (11) eingeführte Schraubendreher (48) in einer axialen Sollposition arretierbar ist, in der sein Drehbetätigungselement (114) zumindest teilweise in die Ausnehmung (13) des Schraubenkopfes (3) hineinragt.

20. Instrumentensatz nach Anspruch 19, **dadurch gekennzeichnet, dass** die Arretiereinrichtung einen aus der Umfangsfläche des Schraubendrehers 48 vorstehende Vorsprung (117) und ein diesen in der Sollposition des Schraubendrehers (48) auf seiner Distalseite radial überlappendes, gegenüber dem Schraubenmanipulator axialfestes Arretierelement umfasst.

21. Instrumentensatz nach Anspruch 20, **dadurch gekennzeichnet, dass** das Arretierelement ein separates, lösbar und axialfest am Schraubenmanipulator (2) fixierbares Arretierelement (AE) umfasst.

22. Instrumentensatz nach Anspruch 21, **dadurch gekennzeichnet, dass** das Arretierelement (AE) eine von einer Radialnut (118) durchsetzte Scheibe (119) ist, wobei die lichte Weite (120) der Radialnut (118) größer ist als die Dicke oder der Durchmesser (124) eines sich distal an den Vorsprung (117) anschließenden Längsabschnitts 125 des Schraubendrehers (48), aber kleiner ist, als die Abmessung des Vorsprungs 117 in einer sich quer zur Mittellängsachse des Schraubendrehers (48) erstreckenden Richtung.

23. Instrumentensatz nach Anspruch 21 oder 22, **gekennzeichnet durch** eine in Radialrichtung wirksame Rastverbindung zwischen der Scheibe (119) und dem Schraubenmanipulator (2).

24. Instrumentensatz nach Anspruch 23, **dadurch gekennzeichnet, dass** aus der proximalen Seite der Scheibe (119) ein federnd gelagertes Rastelement (RE) beispielsweise in Form einer Kugel vorsteht, welches in eine Ringnut (127) auf der distalen Stirnseite des Schiebers (24) bzw. des zweiten Zylinderabschnitts (67) einrastet.

25. Instrumentensatz nach einem der Ansprüche 22 bis 24, **dadurch gekennzeichnet, dass** auf der proximalen Seite der Scheibe 119 ein Hintergriffselement HE angeordnet ist, welches zur
axialen Arretierung des Schraubendrehers (48) mit einer sich quer zur Mittellängsachse des Schraubendrehers erstreckenden Fügerichtung auf den Schraubenmanipulator (2) aufsteckbar ist, und dabei ein Gegenelement (GE) hintergreift.

26. Instrumentensatz nach Anspruch 25, **dadurch gekennzeichnet, dass** das Gegenelement (GE) die zum proximalen Ende des Schraubenmanipulator (2) weisende Nutwand (125) der am zweiten Zylinderabschnitt (67) vorhandenen Ringnut (74) ist.

27. Instrumentensatz nach Anspruch 25 oder 26, **dadurch gekennzeichnet, dass** das Hintergriffselement (HE) ein mit Axialabstand zur proximalen Seite der Scheibe (119) angeordneter U-förmig verlaufender Flansch (128) ist.

28. Instrumentensatz nach einem der vorhergehenden Ansprüche, **dadurch gekennzeichnet, dass** er ein Greifwerkzeug (140) mit einem stabförmigen Schaft (144) aufweist, an dessen proximalem Ende ein zum Festhalten eines Stabs (8) dienendes Greifelement und an dessen anderem Ende ein langgestreckter Handgriff (141) angeordnet ist, wobei der Handgriff (141) so ausgerichtet ist, dass seine Längsrichtung (146) mit der Längsrichtung (147) des Schafts (144) einen Winkel (α) kleiner 180° und größer 90° einschließt.

29. Instrumentensatz nach Anspruch 28, **gekennzeichnet durch** einen Winkel (α) von 100° bis 140°.

30. Instrumentensatz nach Anspruch 29, **gekennzeichnet durch** einen Winkel (α) von 110° bis 130°.

31. Instrumentensatz nach einem der Ansprüche 28 bis 30, **dadurch gekennzeichnet, dass** der Schaft (144) und der Handgriff (141) jeweils in Längsrichtung (146, 147) von einem Hohlraum (148, 149) durchsetzt sind, wobei die Hohlräume an einer Verbindungsstelle (150) von Handgriff und Schaft miteinander verbunden sind, und wobei in den Hohlräumen eine das Greifelement in eine den Stab (8) einklemmende Greifstellung und in eine den Stab frei gebenden Freigabestellung überführende Betätigungseinrichtung (154) angeordnet ist.

32. Instrumentensatz nach Anspruch 31, **dadurch gekennzeichnet, dass** die Betätigungseinrichtung (154) ein axial beweglich im Hohlraum (148) des Handgriffs (141) angeordnetes stabförmiges Antriebselement (155) und ein axial beweglich im Hohlraum (149) des Schafts (144) angeordnetes stabförmiges Abtriebselement (156) umfasst, wobei die an der Verbindungsstelle (150) einander zugewandten Enden des Antriebselements und des Abtriebselements, ein Keilgetriebe bilden.

33. Instrumentensatz nach Anspruch 32, **dadurch gekennzeichnet, dass** eine am Antriebselement (155) oder am Abtriebselement (156) vorhandene Schrägfläche (157) so ausgerichtet ist, dass sie mit der Längsrichtung (146, 147) des jeweiligen Antriebs- bzw. Abtriebselements (155) einen Winkel (β) einschließt, welcher mit einer Abweichung von 20° dem halben von den Längsrichtungen (146, 147) eingeschlossenen Winkel (α) entspricht.

34. Instrumentensatz nach Anspruch 32 oder 31, **dadurch gekennzeichnet, dass** das Antriebselement (155) oder das Abtriebselement (156) von einer Bohrung (158) durchsetzt sind, deren Bohrungswand wenigstens eine Schrägfläche (157a, 157b) bildet, wobei das jeweils andere Element (155, 156) in die Bohrung (158) hinein ragt und mit der wenigstens einen Schrägfläche in Sinne eines Keilgetriebes zusammenwirkt.

35. Instrumentensatz nach Anspruch 34, **dadurch gekennzeichnet, dass** die Bohrung (158) einen kreisrunden Bohrungsquerschnitt hat, wobei das Antriebselement (155) oder das Abtriebselement (156) mit einer Kugel (164) in die Bohrung (158) hinein ragt.

36. Instrumentensatz nach einem der Ansprüche 32 bis 35, **dadurch gekennzeichnet, dass** das Antriebselement (155) ein mit einem Innengewinde (180) des Handgriffs (141) zusammenwirkendes Außengewinde (177) aufweist, wobei das Antriebselement (155) mit einem Überstand (178)aus dem Handgriff (141) heraus ragt, an dem ein Handrad (179) angeordnet ist.

37. Instrumentensatz nach einem der Ansprüche 32 bis 36, **dadurch gekennzeichnet, dass** das Greifelement von der proximalen Stirnseite (186) des Abtriebselements (156) gebildet ist.

38. Instrumentensatz nach Anspruch 37, **dadurch gekennzeichnet, dass** der Schaft (144) an seinem Freiende von einer zur Aufnahme des Stabs (8) dienenden, mit dem Hohlraum (149) verbundenen Bohrung (187) durchsetzt ist, wobei in der Greifstellung das Greifelement bzw. die Stirnseite (186) des Abtriebselements (156) den Stab (8) gegen einen in Distalrichtung weisenden Bereich der Bohrungswandung drückt.

39. Instrumentensatz nach Anspruch 38, **dadurch gekennzeichnet, dass** die proximale Stirnseite (186) des Abtriebselements (156) konkav ausgebildet ist und Teil einer Zylinderfläche ist, die eine zur Oberflächenkrümmung des Stabs (8) komplementäre Krümmung aufweist.

40. Instrumentensatz nach Anspruch 38, **dadurch gekennzeichnet, dass** zur drehfesten Fixierung des Stabs (8) in der Bohrung (187) aus der proximalen Stirnseite (186) des Abtriebselements (156) wenigsten ein Vorsprung (188) heraus ragt.

41. Instrumentensatz nach einem der Ansprüche 38 bis 40, **dadurch gekennzeichnet, dass** die Mittellängsachse (195) der Bohrung (188) in der von den Längsrichtungen (146, 147) des Handgriffs (141) und des Schafts (144) aufgespannten Ebene verläuft.

## Claims

1. Set of instruments for percutaneous spinal column stabilisation by means of rods (8) and pedicle screws (1) having a screw head (3) and a screw shank (4), said pedicle screws (1) having a distally open recess (13) for receiving a rod (8), having a screw manipulator (2), through which a central cavity (11) passes axially and which comprises a cylindrical sleeve (20) and a slide (24), wherein
- the wall of the cylindrical sleeve (20) is divided into two first wall segments (27) by two diametrically opposed, axially running windows (26) opening out to the proximal end face of the sleeve (20),
- the proximal ends of the first wall segments (27) form gripping elements (28) which receive at least one longitudinal section of the screw head (3) between them during screw manipulation, and
- the slide (24) can be moved on the sleeve (20) in the axial direction and is connected to the first wall segments (27) in such a way that it secures these against radial divergence at least in a proximal movement end position (E),
- the slide (24) is a hollow cylinder whose wall is divided into two second wall segments (58) by two diametrically opposed, axially running windows (57) opening out to the proximal end face of the hollow cylinder, said wall segments (58) being guided in an axially displaceable manner into the windows (26) of the sleeve (20) and whose proximal end faces each form a hold-down element (35), **characterised in that**,
- a first or second wall segment (27, 58) has a rib (64) projecting from its side edge which points in the peripheral direction (60) of the screw manipulator (2), said rib (64) extending in the axial direction, and a groove (65) extending in the axial direction is present in each case in the side edges of the respective other wall segment (27, 58), the rib (64) engaging in said groove (65) in an axially displaceable manner with a positive connection that acts in the peripheral direction.

2. Instrument set according to claim 1, **characterised in that** the slide (24) has at least one hold-down element (35) on its proximal end, said hold-down element (35), in the proximal end position of the slide, holding a rod (8) inside the recess (13), said rod (8) being inserted into a distally open recess (13) of the screw head (3).

3. Instrument set according to claim 2, **characterised in that** the at least one hold-down element (35) in the proximal sliding end position (E) of the slide (24) has an axial distance (38) to the end face (25) of the sleeve (20) or the first wall segments (27) which is smaller than the penetration depth (39) with which the screw head (3) protrudes axially into a receiving space (37) which is laterally limited by the gripping elements (28).

4. Instrument set according to claim 3, **characterised in that** the axial distance (38) is smaller than or equal to the difference between the penetration depth (39) and the thread length (17) of an internal thread (16) present in the screw head (3) which is used for fixing the rod (8) with the aid of a fixing screw (5).

5. Instrument set according to claim 2, 3 or 4, **characterised in that** the at least one hold-down element (35) is arranged outside of an imaginary cylinder (44) that runs coaxially to the central longitudinal axis (40) of the sleeve (20), the diameter (45) of which cylinder (44) corresponds to the diameter (46) of the external thread (12) of the fixing screw (5).

6. Instrument set according to claim 5, **characterised in that** the diameter (45) of the imaginary cylinder (44) corresponds to the external diameter (49) of the screw head (3).

7. Instrument set according to one of the preceding claims, **characterised in that** the first wall segments (27) extend over a greater arc length than the second wall segments (58).

8. Instrument set according to one of the preceding claims, **characterised in that** the exterior surfaces of the first and second wall segments (27, 58) extend in a common cylinder enveloping surface.

9. Instrument set according to one of the preceding claims, **characterised in that** the distal ends of the first wall segments (27) are connected to one another by a first cylinder section (66) and the distal ends of the second wall elements (58) are connected to one another by a second cylinder section (67), wherein the inner diameter (68) of the first cylinder section (66) is greater than the outer diameter (69) of the second cylinder section (67).

10. Instrument set according to one of the preceding claims, **characterised in that** an axial movement of the slide (24) relative to the sleeve (20) takes place by means of a worm gear.

11. Instrument set according to claim 10 in conjunction with claim 7, **characterised in that** an external thread (75) projects out from the outer surface of the second wall segments (58), said external thread (75) interacting with an internal thread (76) of an axially fixed adjustment nut (77) which coaxially surrounds the sleeve (20).

12. Instrument set according to claim 11, **characterised in that** a proximal and a distal stop element (pA, dA) project out from the outer surfaces of the first wall segments (27) at an axial distance from one another to axially fix the adjustment nut (77), said stop elements (pA, dA) enclosing and radially overlapping a counter-stop element (GA) which projects radially inward from the inner surface of the adjustment nut (77).

13. Instrument set according to claim 12, **characterised in that** the distal stop element (dA) is formed from the first cylinder section (66), wherein this cylinder section radially extends beyond the first wall segments (27) by means of a shoulder (86).

14. Instrument set according to claim 12 or 13, **characterised in that** the proximal stop element (pA) is a stop ring (87) which encircles the sleeve (20), said stop ring (87) being releasably connected to the first wall segments (27) in an axially fixed manner.

15. Instrument set according to one of claims 12 to 14, **characterised in that** the counter-stop element (GA) is the internal thread (76) of the adjustment nut (77).

16. Instrument set according to one of claims 12 to 15, **characterised in that** the adjustment nut (77) is arranged on the distal end of the sleeve (20).

17. Instrument set according to claim 16, **characterised in that** the adjustment nut (77) encircles at least one longitudinal section of the first cylinder section (66).

18. Instrument set according to one of the preceding claims, **characterised in that** it comprises a rod-shaped screwdriver (48) that can be inserted into the central cavity (11) of the screw manipulator (2), a rotary actuator (114), which can be non-rotatably inserted into the recess (13) of the screw head (3), being arranged on the proximal end of said screwdriver (48).

19. Instrument set according to claim 18, **characterised by** a locking device, with which the screwdriver (48), which has been inserted into the cavity (11), can be locked in an axial target position, in which its rotary actuator (114) protrudes at least partially into the recess (13) of the screw head (3).

20. Instrument set according to claim 19, **characterised in that** the locking device comprises a projection (117) projecting from the peripheral surface of the screwdriver (48) and a locking element which radially overlaps this projection (117) in the target position of the screwdriver (48) on its distal side and is axially fixed with respect to the screw manipulator.

21. Instrument set according to claim 20, **characterised in that** the locking element comprises a separate, releasable locking element (AE) which can be fastened to the screw manipulator (2) in an axially fixed manner.

22. Instrument set according to claim 21, **characterised in that** the locking element (AE) is a disc (119) through which a radial groove (118) passes, wherein the clear width (120) of the radial groove (118) is greater than the thickness or the diameter (124) of a longitudinal section (125) of the screwdriver (48), said longitudinal section (125) being distally connected to the projection (117), but is smaller than the size of the projection (117) in a direction extending transversely to the central longitudinal axis of the screwdriver (48).

23. Instrument set according to claim 21 or 22, **characterised by** a latching connection between the disc (119) and the screw manipulator (2) effective in the radial direction.

24. Instrument set according to claim 23, **characterised in that** a spring-loaded latching element (RE) projects out from the proximal side of the disc (119), for instance in the form of a ball, which latches in a ring groove (127) on the distal end face of the slide (24) or the second cylinder section (67).

25. Instrument set according to one of claims 22 to 24, **characterised in that** a rear engagement element HE is arranged on the proximal side of the disc (119), which can be attached to the screw manipulator (2) in a joining direction extending transversely to the central longitudinal axis of the screwdriver in order to axially lock the screwdriver (48), and here engages behind a counter-element (GE).

26. Instrument set according to claim 25, **characterised in that** the counter-element (GE) is the groove wall (125) of the ring groove (74) present on the second cylinder section (67), said groove wall (125) facing towards the proximal end of the screw manipulator (2).

27. Instrument set according to claim 25 or 26, **characterised in that** the rear-engagement element (HE) is a flange (128) which is arranged with an axial distance to the proximal side of the disc (119) and runs in a U-shaped manner.

28. Instrument set according to one of the preceding claims, **characterised in that** it has a gripping tool (140) having a rod-shaped shaft (144), a gripping element for the purpose of securing a rod (8) being arranged on the proximal end of said shaft (144) and an elongated handle (141) being arranged on the other end thereof, wherein the handle (141) is orientated in such a way that its longitudinal direction (146) forms an angle (α) with the longitudinal direction (147) of the shaft (144) which is smaller than 180° and greater than 90°.

29. Instrument set according to claim 28, **characterised by** an angle (α) of 100° to 140°.

30. Instrument set according to claim 29, **characterised by** an angle (α) of 110° to 130°.

31. Instrument set according to one of claims 28 to 30, **characterised in that** the shaft (144) and the handle (141) each have a cavity (148, 149) passing through them in the longitudinal direction (146, 147), wherein the cavities are connected to each other at a connection point (150) of the handle and shaft, and wherein an actuating device (154) is arranged in the cavities, which transfers the gripping element into a gripping position clamping the rod (8) and into a release position releasing the rod.

32. Instrument set according to claim 31, **characterised in that** the actuating device (154) comprises a rod-shaped input element (155) which is arranged in the cavity (148) of the handle (141) in an axially moveable manner and a rod-shaped output element (156) which is in the cavity (149) of the shaft (144) in an axially moveable manner, wherein the ends of the input element and the output element which face each other at the connection point (150) form a wedge gear.

33. Instrument set according to claim 32, **characterised in that** an inclined surface (157) present on the input element (155) or on the output element (156) is orientated in such a way that it forms an angle (β) with the longitudinal direction (146, 147) of the respective input element (155) or output element (156), said angle (β) corresponding to half of the angle (α) formed by the longitudinal directions (146, 147), with a deviation of 20°.

34. Instrument set according to claim 32 or 31, **characterised in that** the input element (155) or the output element (156) are penetrated by a bore (158), the bore wall thereof forming at least one inclined surface (157a, 157b), wherein the respective other element (155, 156) protrudes into the bore (158) and interacts with the at least one inclined surface in the sense of a wedge gear.

35. Instrument set according to claim 34, **characterised in that** the bore (158) has a circular bore cross-section, wherein the input element (155) or the output element (156) protrudes into the bore (158) with a ball (164).

36. Instrument set according to one of claims 32 to 35, **characterised in that** the input element (155) has an external thread (177) that interacts with an internal thread (180) of the handle (141), wherein the output element (155) protrudes from the handle (141) with an overhang (178), a handwheel (179) being arranged on said handle (141).

37. Instrument set according to one of claims 32 to 36, **characterised in that** the gripping element is formed by the proximal end face (186) of the output element (156).

38. Instrument set according to claim 37, **characterised in that** the shaft (144) is penetrated at its free end by a bore (187) which serves to receive the rod (8) and is connected to the cavity (149), wherein, in the gripping position, the gripping element or the end face (186) of the output element (156) presses the rod (8) against a region of the bore wall which points in the distal direction.

39. Instrument set according to claim 38, **characterised in that** the proximal end face (186) of the output element (156) is formed to be concave and is part of a cylindrical surface, which has a curvature complementary to the surface curvature of the rod (8).

40. Instrument set according to claim 38, **characterised in that** at least one projection (188) protrudes from the proximal end face (186) of the output element (156) in order to non-rotatably fix the rod (8) in the bore (187).

41. Instrument set according to one of claims 38 to 40, **characterised in that** the central longitudinal axis (195) of the bore (188) runs on the plane defined by the longitudinal directions (146, 147) of the handle (141) and the shaft (144).

## Revendications

1. Kit d'instruments pour la stabilisation percutanée de la colonne vertébrale à l'aide de tiges (8) et de vis (1) pédiculaires, qui ont une tête (3), un fût (4) et un creux (13) ouvert distalement de réception d'une tige (8), ayant un manipulateur (2) de vis traversé axialement par une cavité (11) centrale et comprenant un manchon (20) cylindrique et un coulisseau (24), dans lequel
- la paroi du manchon (20) cylindrique est subdivisée en deux premiers segments (27) de paroi par deux fenêtres (26) opposées diamétralement, s'étendant axialement et débouchant dans le côté frontal proximal du manchon (20),
- les extrémités proximales des premiers segments (27) de paroi forment des éléments (28) de prise, qui, lors d'une manipulation de la vis, reçoivent entre eux au moins une partie longitudinale de la tête (3) de la vis et
- le coulisseau (24) peut coulisser dans la direction axiale sur le manchon (20) et être relié ainsi aux premiers segments (27) de paroi, de manière à les empêcher, au moins, dans une position (E) d'extrémité de coulissement proximal, de s'écarter radialement les uns des autres,
- le coulisseau (24) est un cylindre creux, dont la paroi est subdivisée en deux deuxièmes segments (58) de paroi par deux fenêtres (57) opposées diamétralement, s'étendant axialement et débouchant dans le côté frontal proximal du cylindre creux, segments (58), qui sont guidés à coulissement axial dans les fenêtres (26) du manchon (20) et dont les côtés frontaux proximaux forment chacun un élément (35) de maintien vers le bas, **caractérisé en ce que**
- un premier ou un deuxième segment (27, 58) de paroi a une nervure (64), qui est en saillie de son bord latéral, tourné dans la direction (60) périphérique du manipulateur (2) de vis, et qui s'étend dans la direction axiale et, dans les bords latéraux de l'autre segment (27, 58) de paroi, est ménagée respectivement une rainure (65), qui s'étend dans la direction axiale et dans laquelle la nervure (64) pénètre à coulissement axial par une complémentarité de forme efficace dans la direction périphérique.

2. Kit d'instruments suivant la revendication 1, **caractérisé en ce que** le coulisseau (24) a, à son extrémité proximale, au moins un élément (35) de maintien vers le bas, qui, dans la position d'extrémité proximale du coulisseau, maintient une tige (8) insérée dans un creux (13), ouvert distalement, de la tête (3) de la vis, à l'intérieur du creux (13).

3. Kit d'instruments suivant la revendication 2, **caractérisé en ce que** le au moins un élément (35) de maintien vers le bas a, dans la position (E) d'extrémité de coulissement proximal du coulisseau (24), une distance (38) axiale au côté (25) frontal du manchon (20) ou des premiers segments (27) de paroi, qui est plus petite que la profondeur (39) de pénétration, à laquelle la tête (3) de la vis pénètre axialement dans un espace (37) de réception délimité latéralement par les éléments (28) de prise.

4. Kit d'instruments suivant la revendication 3, **caractérisé en ce que** la distance (38) axiale est inférieure ou égale à la différence entre la profondeur (39) de pénétration et la longueur (17) de filet d'un taraudage (16) prévu dans la tête (3) de la vis et servant à immobiliser la tige (8) à l'aide d'une vis (5) d'immobilisation.

5. Kit d'instruments suivant la revendication 2, 3 ou 4, **caractérisé en ce que** le au moins un élément (35) de maintien vers le bas est disposé à l'extérieur d'un cylindre (44) imaginaire, qui s'étend coaxialement à l'axe (40) longitudinal médian du manchon (20) et dont le diamètre (45) correspond au diamètre (46) du filet (12) de la vis (5) d'immobilisation.

6. Kit d'instruments suivant la revendication 5, **caractérisé en ce que** le diamètre (45) du cylindre (44) imaginaire correspond au diamètre (49) extérieur de la tête (3) de la vis.

7. Kit d'instruments suivant l'une des revendications précédentes, **caractérisé en ce que** les premiers segments (27) de paroi s'étendent sur une longueur d'arc plus grande que les deuxièmes segments (58) de paroi.

8. Kit d'instruments suivant l'une des revendications précédentes, **caractérisé en ce que** les surfaces extérieures des premiers et deuxièmes segments (27, 58) de paroi s'étendent suivant une surface d'enveloppe cylindrique commune.

9. Kit d'instruments suivant l'une des revendications précédentes, **caractérisé en ce que** les extrémités distales des premiers segments (27) de paroi sont reliées entre elles par un premier tronçon (66) de cylindre et les extrémités distales des deuxièmes éléments (58) de paroi par un deuxième tronçon (67) de cylindre, le diamètre (68) intérieur du premier tronçon (66) de cylindre étant plus grand que le diamètre (69) extérieur du deuxième tronçon (67) de cylindre.

10. Kit d'instruments suivant l'une des revendications précédentes, **caractérisé en ce qu'**un déplacement axial du coulisseau (24), par rapport au manchon (20), s'effectue par une transmission à vis.

11. Kit d'instruments suivant la revendication 10 en liaison avec la revendication 7, **caractérisé en ce que**, de la surface extérieure des deuxièmes segments (58) de paroi, fait saillie un filetage (75), qui coopère avec un taraudage (76) d'un écrou (77) de réglage immobilisé axialement et entourant coaxialement le manchon (20).

12. Kit d'instruments suivant la revendication 11, **caractérisé en ce que**, pour immobiliser axialement l'écrou (77) de réglage, font saillie des surfaces extérieures des premiers segments (27) de paroi à distance axiale l'un de l'autre un élément (pA) proximal de butée et un élément (dA) distal de butée, qui se chevauchent radialement et incluent entre eux un élément (GA) antagoniste de butée en saillie, vers l'intérieur radialement, de la surface intérieure de l'écrou (77) de réglage.

13. Kit d'instruments suivant la revendication 12, **caractérisé en ce que** l'élément (dA) distal de butée est formé par le premier tronçon (66) de cylindre, celui-ci dépassant radialement, par un épaulement (86), les surfaces extérieures des premiers segments (27) de paroi.

14. Kit d'instruments suivant la revendication 12 ou 13, **caractérisé en ce que** l'élément (pA) proximal de butée est une bague (87) de butée, qui entoure le manchon (20) et qui est reliée de manière amovible, en étant fixe axialement, aux premiers segments (27) de paroi.

15. Kit d'instruments suivant l'une des revendications 12 à 14, **caractérisé en ce que** l'élément (GA) antagoniste de butée est le taraudage (76) de l'écrou (77) de réglage.

16. Kit d'instruments suivant l'une des revendications 12 à 15, **caractérisé en ce que** l'écrou (77) de réglage est disposé à l'extrémité distale du manchon (20).

17. Kit d'instruments suivant la revendication 16, **caractérisé en ce que** l'écrou (77) de réglage prend au moins un tronçon longitudinal du premier tronçon (66) de cylindre.

18. Kit d'instruments suivant l'une des revendications précédentes, **caractérisé en ce qu'**il comprend un tournevis (48), qui peut être introduit dans la cavité (11) centrale du manipulateur (2) de vis et à l'extrémité proximale duquel est disposé un élément (114) d'actionnement en rotation, pouvant être enfiché d'une manière fixe en rotation dans le creux (13) de la tête (3) de la vis.

19. Kit d'instruments suivant la revendication 18, **caractérisé par** un dispositif de blocage, par lequel le tournevis (48) inséré dans la cavité (11) peut être bloqué dans une position axiale de consigne, dans laquelle son élément (114) d'actionnement en rotation pénètre, au moins en partie, dans le creux (13) de la tête (3) de la vis.

20. Kit d'instruments suivant la revendication 19, **caractérisé en ce que** le dispositif de blocage comprend une saillie (117) en saillie de la surface périphérique du tournevis (48) et, le chevauchant radialement sur son côté distal dans la position de consigne du tournevis (48), un élément de blocage fixe axialement par rapport au manipulateur de vis.

21. Kit d'instruments suivant la revendication 20, **caractérisé en ce que** l'élément de blocage comprend un élément (AE) de blocage distinct, mobile et pouvant être immobilisé de manière fixe axialement sur le manipulateur (2) de vis.

22. Kit d'instruments suivant la revendication 21, **caractérisé en ce que** l'élément (AE) de blocage est une rondelle (119) traversée par une rainure (118) radiale, la dimension (120) intérieure de la rainure (118) radiale étant plus grande que l'épaisseur ou que le diamètre (124) d'un tronçon (125) longitudinal, se raccordant distalement à la saillie (117), du tournevis (48), mais plus petite que la dimension de la saillie (117) dans une direction s'étendant transversalement à l'axe longitudinal médian du tournevis (48).

23. Kit d'instruments suivant la revendication 21 ou 22, **caractérisé par** une liaison par encliquetage, efficace dans la direction radiale, entre la rondelle (119) et le manipulateur (2) de vis.

24. Kit d'instruments suivant la revendication 23, **caractérisé en ce que**, du côté proximal de la rondelle (119), fait saillie un élément (RE) d'encliquetage monté élastiquement, par exemple sous la forme d'une bille, qui s'encliquette dans une rainure (127) annulaire du côté frontal distal du coulisseau (24) ou du deuxième tronçon (67) de cylindre.

25. Kit d'instruments suivant l'une des revendications 22 à 24, **caractérisé en ce que**, du côté proximal de la rondelle (119), est disposé un élément (HE) de prise par l'arrière, qui, pour le blocage axial du tournevis (48), peut être enfiché sur le manipulateur (2) de vis dans une direction de jonction s'étendant transversalement à l'axe longitudinal médian du tournevis et ainsi prendre par derrière l'élément (GE) antagoniste.

26. Kit d'instruments suivant la revendication 25, **caractérisé en ce que** l'élément (GE) antagoniste est la paroi (125), tournée vers l'extrémité proximale du manipulateur (2) de vis, de la rainure (74) annulaire présente sur le deuxième tronçon (67) de cylindre.

27. Kit d'instruments suivant la revendication 25 ou 26, **caractérisé en ce que** l'élément (HE) de prise par l'arrière est bride (128) s'étendant en forme de U, à distance axiale du côté proximal de la rondelle (119).

28. Kit d'instruments suivant l'une des revendications précédentes, **caractérisé en ce qu'**il a un outil (140) de préhension ayant un fût (144) en forme de barre, à l'extrémité proximale duquel est monté un élément de préhension servant à fixer une tige (8) et à l'autre extrémité duquel est montée une poignée (141) s'étendant en longueur, la poignée (141) étant orientée de manière à ce que sa direction (146) longitudinale fasse un angle (α) plus petit que 180° et plus grand que 90° avec la direction (147) longitudinale du fût (144).

29. Kit d'instruments suivant la revendication 28, **caractérisé par** un angle (α) de 100° à 140°.

30. Kit d'instruments suivant la revendication 29, **caractérisé par** un angle (α) de 110° à 130°.

31. Kit d'instruments suivant l'une des revendications 28 à 30, **caractérisé en ce que** le fût (144) et la poignée (141) sont traversés chacun dans la direction (146, 147) longitudinale par une cavité (148, 149), les cavités communiquant entre elles par un point (150) de communication de la poignée et du fût et dans lequel il est monté, dans les cavités, un dispositif (154) d'actionnement faisant passer l'élément de préhension, dans une position de préhension, enserrant la tige (8) et, dans une position de libération, libérant la tige (8).

32. Kit d'instruments suivant la revendication 31, **caractérisé en ce que** le dispositif (154) d'actionnement comprend un élément (155) menant, en forme de barre, mobile axialement dans la cavité (148) de la poignée (141) et un élément (156) mené en forme de barre, mobile axialement dans la cavité (149) du fût (144), les extrémités de l'élément menant et de l'élément mené, tournées l'une vers l'autre au point (150) de communication, formant une transmission à clavette.

33. Kit d'instruments suivant la revendication 32, **caractérisé en ce qu'**une surface (157) inclinée présente sur l'élément (155) menant ou sur l'élément (156) mené est orientée de manière à faire avec la direction (146, 147) longitudinale de l'élément menant ou de l'élément (155) mené un angle (β), qui correspond avec un écart de 20° à la moitié de l'angle (α) entre les directions (146, 147) longitudinales.

34. Kit d'instruments suivant la revendication 32 ou 31, **caractérisé en ce que** l'élément (155) menant ou l'élément (156) mené sont traversés par un trou (158), dont la paroi forme au moins une surface (157a, 157b) inclinée, l'autre élément (155, 156) pénétrant dans le trou (158) et coopérant avec la au moins une surface inclinée au sens d'une transmission à clavette.

35. Kit d'instruments suivant la revendication 34, **caractérisé en ce que** le trou (158) a une section transversale circulaire, l'élément (155) menant ou l'élément (156) mené pénétrant, par une bille (164), dans le trou (158).

36. Kit d'instruments suivant l'une des revendications 32 à 35, **caractérisé en ce que** l'élément (155) menant a un filetage (177) coopérant avec un taraudage (180) de la poignée (141), l'élément (155) menant dépassant par un dépassement (178) de la poignée (141), sur lequel est monté un volant (179).

37. Kit d'instruments suivant l'une des revendications 32 à 36, **caractérisé en ce que** l'élément de préhension est formé par le côté (186) frontal proximal de l'élément (156) mené.

38. Kit d'instruments suivant la revendication 37, **caractérisé en ce que** le fût (144) est traversé à son extrémité libre par un trou (187) servant à la réception de la tige (8) et communiquant avec la cavité (149), l'élément de préhension ou le côté (186) frontal de l'élément (156) mené poussant, dans la position de préhension, la tige (8) sur une partie, tournée dans la direction distale, de la paroi du trou.

39. Kit d'instruments suivant la revendication 38, **caractérisé en ce que** le côté (186) frontal proximal de l'élément (156) mené est concave et fait partie d'une surface de cylindre, qui a une courbure complémentaire de la courbure de surface de la tige (8).

40. Kit d'instruments suivant la revendication 38, **caractérisé en ce que**, pour l'immobilisation fixe en rotation de la tige (8) dans le trou (187), au moins une saillie (188) dépasse du côté (186) frontal proximal de l'élément (156) mené.

41. Kit d'instruments suivant l'une des revendications 38 à 40, **caractérisé en ce que** l'axe (195) longitudinal médian du trou (188) s'étend dans le plan passant par les directions (146, 147) longitudinales de la poignée (141) et du fût (144).
